(19)

Europäisches
Patentamt
European
Patent Office
Office européen
des brevets

(11) **EP 3 334 413 B1**

(12) **EUROPEAN PATENT SPECIFICATION**

(45) Date of publication and mention
of the grant of the patent:
**18.05.2022 Bulletin 2022/20**

(21) Application number: **16834701.1**

(22) Date of filing: **11.08.2016**

(51) International Patent Classification (IPC):
*A61K 9/00* *(2006.01)*          *A61F 6/14* *(2006.01)*
*A61K 9/52* *(2006.01)*          *A61L 29/16* *(2006.01)*
*A61M 25/10* *(2013.01)*         *A61M 31/00* *(2006.01)*

(52) Cooperative Patent Classification (CPC):
(C-Sets available)
**A61F 6/08; A61F 6/14; A61F 6/142; A61L 29/126;
A61L 29/145; A61L 29/16; A61M 31/002;
A61P 15/18; A61P 25/28; A61P 31/18; C08L 75/04;**
A61K 9/127; A61M 25/0017; Y02W 10/37     (Cont.)

(86) International application number:
**PCT/DK2016/050270**

(87) International publication number:
**WO 2017/025104 (16.02.2017 Gazette 2017/07)**

(54) **A DELIVERY DEVICE**

ABGABEVORRICHTUNG

DISPOSITIF D'ADMINISTRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.08.2015 DK 201570520**

(43) Date of publication of application:
**20.06.2018 Bulletin 2018/25**

(73) Proprietor: **PTT Holding Aps
2800 Lyngby (DK)**

(72) Inventors:
• **THOMSEN, Peter Theilade
2800 Kgs. Lyngby (DK)**
• **ALM, Martin
DK-2670 Greve (DK)**

(74) Representative: **Plougmann Vingtoft a/s
Strandvejen 70
2900 Hellerup (DK)**

(56) References cited:
**EP-A1- 2 745 828      EP-A2- 0 347 145
EP-B1- 1 225 940      WO-A1-2009/096822
WO-A1-2013/075724    WO-A2-2005/055972**

WO-A2-2008/039920     CN-A- 101 733 051
US-A- 4 283 325          US-A- 5 783 633
US-A1- 2014 309 598    US-A1- 2015 157 561
US-B1- 6 331 578

• SHIVASHANKAR, M. ET AL.: 'A REVIEW ON
INTERPENETRATING POLYMER NETWORK'
INTERNATIONAL JOURNAL OF PHARMACY
AND PHARMACETUICAL SCIENCES vol. 4, no.
SUPPL., 2012, pages 1 - 7, XP055181314
• DOLLASE, T. ET AL.: 'Crystallization of PDMS:
The effect of physical and chemical crosslinks'
EUROPHYSICS LETTERS vol. 60, no. 3, 2002,
pages 390 - 396, XP009508613
• MURAYAMA, S. ET AL.: 'Hydrophobic and
hydrophilic interpenetrating polymer networks
composed of polystyrene and
poly(2-hydroxyethyl methacrylate): 1. PS-
PHEMA sequential IPNs synthesized in the
presence of a common solvent' POLYMER vol.
34, no. 13, 1993, pages 2845 - 2852, XP022836203

• MURAYAMA, S. ET AL.: 'Hydrophobic and hydrophilic interpenetrating polymer networks composed of polystyrene and poly(2-hydroxyethyl methacrylate): 2. Gradient composition in the IPNs synthesized by photopolymerization' POLYMER vol. 34, no. 18, 1993, pages 3893 - 3898, XP001179755

(52) Cooperative Patent Classification (CPC): (Cont.)

C-Sets
A61L 29/126, C08L 33/10;
A61L 29/126, C08L 75/04;
A61L 29/126, C08L 83/04;
C08L 75/04, C08L 33/00

**Description**

**TECHNICAL FIELD**

The invention according to the appended claims 1 to 15 relates to a delivery device suitable for delivering a chemical substance, e.g. a medical device such as a catheter, a microcapsule, an implantable capsule or a P-ring, or a delivery device for use in the construction industries e.g. in the form of microcapsules comprising antifouling agent for marine paint.

**BACKGROUND ART**

A plurality of methods for delivering chemical substances such as chemical compounds or compositions is known in the art. In recent years large effort has been applied in developing delivery devices in the form of a polymer matrix, in particular hydrogels, wherein the chemical compound for release embedded in the matrix.

A study by Garcia et al. "5-Fluorouracil trapping in poly(2-hydroxyethyl methacrylate-co-acrylamide) hydrogels; in vitro drug delivery studies", European Polymer Journal 36 (2000) 111-122, describes such delivery devices based on hydrogels where the 5-Fluorouracil was trapped in the gel during its polymerization. In Ferreira et al. "Evaluation of poly(2-hydroxyethyl methacrylate) gels as drug delivery systems at different pH values", International Journal of Pharmaceuticals 194 (2000) 169-180, the influence of the pH value during loading of Salicylic acid in PHEMA gel was tested. The loading was performed by immersing in a liquid comprising the salicylic acid at various pH values.

Karlgard et al. "In vitro uptake and release studies of ocular pharmaceutical agents by silicon-containing and p-HEMA hydrogel contact lens materials", International Journal of Pharmaceuticals 257 (2003) 141-151 discloses a study of the uptake and release of different chemical compounds in different types of contact lenses. The loading was performed by soaking in a liquid containing the chemical compound. It was found that both the uptake and the release were relatively rapid.

Santos et al. "Poly(hydroxyethyl methacrylate-co-methacrylated-p-cyclodextrin) hydrogels: Synthesis, cytocompatability, mechanical properties and drug loading/release properties" ScienceDirect, Acta Biomaterialia 4 (2008) 745-755, describes loading/release properties of hydrocortisone and acetazolamide in the hydrogel. The loading was performed by immersing in liquid containing the drug. It was found that a slow drug release could be obtained.

WO 2005/055972 discloses a drug delivery device comprising a polymeric matrix and a drug distributed within the matrix. The device is essentially free of solvents in particular organic solvents for the drug. The matrix may be an interpenetrating polymer network (IPN) of a large number of different polymer materials.

The drug delivery device is produced by loading the matrix with a drug using a drug carrier in the form of a gas, a supercritical fluid, a high pressure liquid, or a dense gas-like fluid. A preferred drug carrier includes $CO_2$ in a liquid and/or supercritical state. No specific methods are described.

CN103623497A describes a drug delivery balloon dilating catheter of a double-layer balloon structure. The drug delivery balloon dilating catheter comprises a pressurizing hole-free inner balloon body and an outer balloon body with a drug-permeable micro-hole permeable membrane. A similar balloon catheter is described in US 5569184 where the inflating balloon is surrounded by a delivery balloon with delivery ports or of inherently permeable fabrics such that drug introduced into the interspace between the inflating balloon and the delivery balloon is immediately delivered via physical holes provided by the fabrics or the delivery ports.

CN101733051 describes a method of preparing PDMS/PHEMA IPN hollow microcapsules by a method where a mixture of PDMS, TEOS, stannous octoate and toluene is dispersed into PVA solution and cured to form microspheres of a first PDMS network. The first PDMS network spheres are hereafter soaked in a mixture of toluene, crosslinking agent and HEMA for some hours and crosslinked. It is considered that the spheres may be used for drug delivery.

US2014309598A describes an osmotically active intravaginal delivery system for the controlled release of therapeutically active substances into the vaginal cavity. The osmotically active vaginal delivery system has a body which comprises at least one compartment comprising a composition of one or more therapeutically active substances and at least one compartment comprising an osmotically composition capable of interacting with water and aqueous biological fluids to create a concentration gradient against the exterior fluid or to swell or expand to create osmotic pressure and thereby providing a pressure to release the active substances via a passageway extending from the compartment comprising the composition of one or more therapeutically active substances to the outer surface of the body.

US2012080378A describes forward osmosis membranes having a hydrophilic support layer and a polyamide rejection layer in a thin film composite membrane. Preferred support layer materials include aramid polymers and PVDF. A woven or non-woven mesh can be incorporated into the support layer to improve the handling properties of the membrane. Flat sheet and hollow fiber configurations are possible. Antifouling techniques are provided. The polyamide layer can be formed on the hydrophilic support layer by interfacial polymerization. Applications include forward osmosis and pressure retarded osmosis, such as industrial product and/or waste concentration, hydration bags, energy/pressure generation, and controlled delivery of chemicals (e.g. for pharmaceutical applications).

**DISCLOSURE OF INVENTION**

It is an object of the invention to provide a new type of delivery device suitable for delivering a chemical substance which can be applied for controlled release over a relatively long period of time and which is both safe and can dose a chemical substance with high accuracy.

In an embodiment, it is an object of the invention to provide a delivery device which can be applied for delivering chemical substances over a relatively long period of time, in a flexible manner and/or with a desired release profile, such as a 0-order kinetic release profile.

In an embodiment, it is an object of the invention to provide a delivery device which can be applied for delivering chemical substances without the delivery device comprises undesired organic solvents, which is practically all organic solvents with ethanol as an exception.

These and other objects have been solved by the invention as defined in the claims and as described herein below.

It has been found that the invention and embodiments thereof have a number of additional advantages, which will be clear to the skilled person from the following description.

The delivery device of the invention has been found to be suitable for many types of applications where delivery of chemical substances is desired. The delivery device provides a new concept for delivery of drugs and other chemical substances where the delivery device can be designed to have desired release profile or profiles for example constant release, persistent release, zero order release and/or sustained release.

The delivery device comprises a closed cavity. The cavity is defined by an innermost wall surface, wherein at least a section of the inner wall surface constitutes an inner surface of a delivery membrane. The delivery membrane comprises an interpenetrating polymer network (IPN) substrate comprising a host polymer and a guest polymer, where the guest polymer is interpenetrating the host polymer to form continuous pathways within the host polymer.

The term "closed cavity" means herein that the delivery device is configured for holding an amount of liquid such as water within the cavity without leaking the liquid. The delivery device may comprise a closable opening or may be adapted for penetration for filling the chemical substance into the cavity. This will be described in further detail below.

The section of the inner wall surface that constitutes the inner surface of the delivery membrane may be any portion of the inner wall surface, such as the entire inner wall surface or merely a smaller part of the inner wall surface, such as about 1 % or more, such as 10 % or more, such as 25 % or more, such as 50 % or more of the inner wall surface. Interpenetrating polymer networks are well known in the art and further information about interpenetrating polymer networks and how such networks can be provided is for example described in US2015038613, WO 2005/055972 and/or WO 2013/075724.

Any term used in singular form should also be interpreted to include the plural form of the term, unless otherwise specified.

The term "substantially" should herein be taken to mean that ordinary product variances and tolerances are comprised.

In an embodiment, the delivery membrane has an outer surface opposite to the inner surface of the delivery membrane. The distance between the outer surface and inner surface defines the thickness of the delivery membrane. Advantageously, the continuous pathways of the guest polymer within the host polymer extend from the inner surface to the outer surface of the delivery membrane. Thereby the chemical substance can migrate through the delivery membrane via the interpenetrating pathways. The skilled person can design the release profile of the delivery membrane by adjusting the amount of guest polymer in the host polymer and by designing the formation of the pathways. By preparing the host polymer with a surplus of residuals that can be extracted e.g. by liquid, dense or supercritical $CO_2$, the skilled person can obtain a desired interpenetrating polymer network structure of the host polymer. In an embodiment the delivery membrane may on its inner and/or outer surface comprise a coating of a material identical to the material of the guest polymer. Thereby the release of the chemical substance can be increased.

In an embodiment the host polymer is prepared with a surplus of residuals that can be extracted by an organic solvent that swells the host polymer without dissolving it. Afterwards undesired remaining's of organic solvent may be extracted e.g. using ethanol which is usually an acceptable solvent.

The delivery membrane can in principle have any thickness, depending on the desired release profile. In principle the chemical substance will migrate through the guest polymer pathways of the delivery membrane by the shortest route, but by having a delivery membrane with a varying thickness, the release profile may be shaped for example such that when the chemical substance in the cavity is reduced due to migration into and through the membrane for release of the substance at the outer surface of the membrane, the membrane will still maintain a certain amount of chemical substance and thereby the release profile can be relatively constant over an increased period of time in particular where the membrane is relatively thick. Thus, the thicker the membrane the longer a substantially constant release profile may be obtained. In an embodiment the chemical substance also migrates through or via the surface of the host polymer. In another embodiment there is substantially no migration of the chemical substance through the host polymer. In an embodiment where the chemical substance comprises two or more different components, one of these components primarily migrates through the guest polymer and another of the components primarily migrates through the host polymer.

Depending on the substances to be released, the substances usually also have certain solvability in the guest and/or the host matrix and intra molecular forces between the guest polymer/the host polymer, and a selected substance to be released can also be applied to shape the release profile. In an embodiment the host polymer and/or guest polymer is selected in relation to the substance(s) to be released a to have a work of adhesion (Whc) at 25 °C of at least about 0 J/m2 where the loading fluid is water preferably as described in US2014303263.

Advantageously at least a section of the delivery membrane has a thickness from 1 $\mu$m to 1 cm, preferably from 10 $\mu$m to 1 mm. A too thin delivery membrane may have too little mechanical toughness whereas the thicker the membrane the longer it will take the chemical substance to migrate through the delivery membrane. Advantageously the delivery membrane is resilient and may be stretched when the cavity is filled with the chemical compound(s), and in this embodiment the thickness in non-stretched state may be selected to be I the larger end of the before mentioned ranges since the thickness will decrease as the delivery membrane is stretched.

The delivery membrane can be uniform or non-uniform. In an embodiment, the delivery membrane has a substantially uniform thickness. In general, a uniform thickness of the delivery membrane is simpler to design and produce.

The host polymer can in principle be any kind of polymer which is not dissolved by the chemical substance e.g. aqueous chemical substance and which has a sufficient mechanical toughness for the intended use of the delivery device. In general, the purpose of the host polymer is to ensure sufficient mechanical toughness of the delivery membrane.

Generally, it is desired that the strength of the membrane is mainly or fully provided by the host polymer. Thus in an embodiment the host polymer has a tear strength substantially identical to the membrane.

In an embodiment the host polymer is a cross-linked polymer, such as a cross-linked elastomer e.g. thermoplastic elastomer (TPE), polyolefin elastomer (POE), polyurethane (PU), rubber e.g. latex rubber, silicone or any combinations thereof.

In an embodiment, the host polymer is physically cross-linked, the host polymer is preferably a physically cross-linked TPE. The physically cross-linked TPE comprises physically cross-linked stabilizing domains that are reversible, and can be reformed e.g. by heat or ion-exchange. The stabilizing domains may be non-crystalline or crystalline.

In an embodiment, the host polymer is chemically cross-linked, the host polymer is preferably covalently cross-linked.

In an embodiment, the host polymer is cross-linked by ionic bonds.

In an embodiment the rubber is natural rubber or synthetic rubber, such as a vulcanized polymer of isoprene, the rubber is preferably a silicone rubber or cross-linked Polyurethane.

In an embodiment, the host polymer is an elastomer. Suitable elastomers include, thermoplastic elastomers (TPEs) polyolefin elastomers (POEs), thermoplastic polyurethane (TPU), rubber e.g. latex rubber, silicones and/or any combinations thereof. Elastomeric host polymers are particularly preferred in embodiments where the delivery membrane should have elastomeric properties e.g. where the delivery membrane is a balloon or a part of a balloon of a catheter.

In a preferred embodiment, the host polymer comprises thermoplastic polyurethane (TPU).

Suitable TPUs include for example the TPUs marketed by Lubrizol under the tradenames Carbothane[tm], Isoplast®, Pellethane®, Tecoflex[tm], Tecophillic[tm] and Tecothane[tm].

TPU is a linear segmented block copolymer composed of hard and soft segments. The hard segment can be either aromatic or aliphatic. Aromatic TPUs are based on isocyanates such as MDI while aliphatic TPU's are based on isocyanates like H12 MDI. When these isocyanates are combined with short-chain diols, they become the hard block. Normally it is aromatic, but when colour and clarity retention in sunlight exposure is a priority, an aliphatic hard segment is often used.

TPU and compounds comprising TPU are very suitable. It is relatively simple to process and can contain large amount of guest polymer. Further TPU is highly elastic and has high resilience. The TPU comprises soft segments, which advantageously can be of polyether or polyester type. The TPU is preferably polyether-based.

In an embodiment, the host polymer comprises at least 10 %, such as at least 20 %, such as at least 40 %, such as at least 60 % by mass of the host polymer with a backbone consisting of Si and O atoms or consisting of Si atoms, the host polymer preferably comprises poly(dimethyl siloxane), poly(methylphenyl siloxane), fluorosilicone rubber, silicone esters, polysiloxanes, polysilanes, polychlorosilanes, polyalkoxysilanes, polyaminosilanes, polysilanes, polydialkylsiloxanes, polysiloxanes containing at least one phenyl substituent, vinyl-functionalized silicone, partially or fully fluorinated silicone or a mixture of two or more of the mentioned silicones.

In a preferred embodiment the host polymer comprises silicone.

It has been found, that the structure of the host polymer may have large influence on the release profile and in particular to obtain a desired release profile. In an embodiment, the host polymer has a structure of a network of strand shaped filaments comprising a plurality of intrastrand pathways. Such host polymer structure of a network of strand shaped filaments comprising a plurality of intrastrand pathways may for example be obtained by preparing the host polymer with a surplus of residuals prior to crosslinking and after crosslinking removing at least a part of such residuals e.g. by supercritical fluid extraction or near supercritical fluid extraction e.g. using $CO_2$ as extraction fluid. In an embodiment the extraction of residuals from the host polymer is performed using dense $CO_2$ gas extraction.

In an embodiment, the host polymer is prepared with a surplus of residuals that can be extracted by an organic solvent that swells the host polymer without dissolving it. Afterwards undesired remaining's of organic solvent may be extracted e.g. using ethanol which is usually an acceptable solvent. The organic solvent may be selected in depends of its ability to swell the host polymer and the time allowed for the swelling may also be used to regulate the removal of residuals from the host polymer.

In an embodiment, the host polymer has a structure of a network of substantially flat strand shaped filaments. The phrase "substantially flat strand shaped filaments" means herein that the strands has a largest cross sectional dimension (perpendicular to a length dimension of the strand) which is much larger, such as at least 100 % larger, such as from about 200 % to 1000 % larger than a shortest cross sectional dimension. The largest cross sectional dimension may be substantially perpendicular to the shortest cross sectional dimension.

In an embodiment the intrastrand pathways has a volume of at least about 30 % of the total volume of the delivery membrane, such as at least about 40 %, such as at least about 50 %, such as at least about 60 % of the total volume of the delivery membrane. The intrastrand pathways volume may for example be determined visually by freezing the host polymer and observing the structure in a microscope, such as a scanning electron microscope (SEM). In an embodiment, the intrastrand pathways volume may for example be determined by weight by determine the weight of the host polymer prior to residual extraction and after residual extraction.

The volume of the intrastrand pathways may be used for designing the release profile. It is believed, that he larger the intrastrand pathways volume the higher amount of drug may pass through the delivery membrane.

In an embodiment the host polymer and/or the guest polymer is electrically conductive.

In an embodiment, the host polymer and the guest polymer have matching refractive index for at least one wavelength, such as for at least one wavelength in the range from 400 nm to 2 $\mu$m, such as in the visible range.

In an embodiment, the host polymer has a higher refractive index than the guest polymer.

In an embodiment, the host polymer and the guest polymer have substantially identical refractive index.

The host polymer may for example have a refractive index of from about 1.33 to about 1.5 and the guest polymer may for example have a refractive index of from about 1.1 to about 1.5. The guest polymer is selected to be suitable for the chemical substance to migrate in the guest polymer pathways. Where the chemical substance is alcohol-soluble, the guest polymer should advantageously have a high alcohol swelling property. The guest polymer may e.g. be an alcogel or a dried alcogel.

Advantageously, the chemical substance is soluble in aqueous media and the guest polymer has a high water swelling property.

In an embodiment, the guest polymer comprises a gel selected from hydrogel, aerogel, xerogel or any combinations thereof.

Where a dry gel is used the dry gel will be re-moistured either by a user prior to using the delivery device, or it will be re-moistured during use by liquid from the liquid in which the chemical substance is dissolved and/or by mucosa liquid (bodily fluids).

In an embodiment, the guest polymer comprises a homopolymer, preferably polymerized from an acrylate monomer or a vinyl polymer, more preferably n-vinyl pyrolidone (nVP), styrene; oxygen-, phenyl-, amino- and nitrogen-containing acrylic and methacrylic derivatives, e.g. acrylic esters, acrylic acids, methacrylic acid and - esters, alkyl and hydroxyalkyl acrylates and methacrylates; functionalized methacrylates such as 2-hydroxyethyl methacrylate (HEMA), glycerol monomethacrylate (GMMA), heptaflurobutyl acrylate (HFBA), 2-methacryloyloxyethyl phosphorylcholine (MPC) and [2-(methacryloyloxy)ethyl]-dimethyl-(3-sulfopropyl)-ammonium hydroxide (Betain); alkyl substituted acrylates and methacrylates such as methyl methacrylate (MMA), ethyl methacrylate (EMA), butyl methacrylate (BMA), dodecyl methacrylate (DMA); PEGylated (meth)actylates such as poly(ethylene glycol) methyl ether methacrylate (PEGMEMA) and poly(ethylene glycol) methyl ether acrylate (PEGMEA); substituted β- and y-lactones, lactic acid monomers; carbohydrides and fluorinated monomers; urethanes; mono- and di-functional alcohols; carboxylic acids; amines; isocyanates; epoxides; aromatics carrying alkyl group(s); sulfonated aromatics, aromatic resins; imidazole; imidazol derivatives; zwitterionic monomers; pyrazoles; quaternary ammonium monomers and any combinations thereof.

Examples of suitable homopolymers also include Nipam (N-Isopropylacrylamide) and pectin.

In an embodiment, the guest polymer comprises a copolymer, preferably polymerised from monomers comprising silanes, e.g. tetraethylorthosilicate or tetraethoxysilane (TEOS), an acrylate monomer or a vinyl polymer, more preferably n-vinyl pyrolidone (nVP), styrene; oxygen-, phenyl, amino and nitrogen-containing acrylic and methacrylic derivatives, e.g. acrylic esters, acrylic acids, methacrylic acid and -esters, alkyl and hydroxyalkyl acrylates and methacrylates; functionalized methacrylates such as 2-hydroxyethyl methacrylate (HEMA), glycerol monomethacrylate (GMMA), heptaflurobutyl acrylate (HFBA), 2-methacryloyloxyethyl phosphorylcholine (MPC) and [2-(methacryloyloxy)ethyl]-dimethyl-(3-sulfopropyl)-ammonium hydroxide (Betain); alkyl substituted acrylates and methacrylates such as methyl methacrylate (MMA), ethyl methacrylate (EMA), butyl methacrylate (BMA), dodecyl methacrylate (DMA); PEGylated (meth)actylates such as poly(ethylene glycol) methyl ether methacrylate (PEGMEMA) and poly(ethylene glycol) methyl ether acrylate (PEGMEA); substituted β- and γ-lactones, lactic acid monomers; carbohydrides and fluorinated monomers; urethanes; mono- and di-functional alcohols; carboxylic acids; amines; isocyanates; epoxides; aromatics carrying alkyl group(s); sulfonated aromatics: aromatic resins; imidazole; imidazol derivatives; zwitterionic monomers; pyrazoles; quaternary ammonium monomers and any combinations thereof.

Copolymers mean polymers that are obtained by copolymerization of two or more monomer species.

In an embodiment the copolymer guest polymer is a bipolymer having two monomer species.

In an embodiment the copolymer guest polymer is a terpolymer obtained from three monomer species.

In an embodiment, the copolymer guest polymer is a quaterpolymer obtained from four monomer species.

In an embodiment the guest polymer comprises poly(2-hydroxyethyl methacrate) (PHEMA), preferably the guest polymer comprises a copolymer of poly(2-hydroxyethyl methacrate) (PHEMA) and PEGMEA.

In an embodiment, the guest polymer has a max. water swelling at 25°C of about 10 - 10000 % by mass of its dry mass, such as of about 10 - 5000 % by mass of its dry mass, such as of about 10 - 500 % by mass of its dry mass, such as of about 100 - 500 % by mass of its dry mass.

In an embodiment, the guest polymer has a max. water swelling at 25°C of its dry mass which is higher than the max. water swelling at 25°C of the host polymer.

In an embodiment the guest polymer has a max. water swelling at 25°C of its dry mass which is equal to or lower than the max. water swelling at 25°C of the host polymer.

In an embodiment, the delivery membrane has a max. water swelling at 25°C of about 5 - 5000 % by mass of its dry mass, such as of about 10 - 1000 % by mass of its dry mass, such as of about 10 - 500 % by mass of its dry mass, such as of about 10 - 40 % by mass of its dry mass.

Depending on the specific requirements which are desired to be met by the delivery device to be provided and the chemical substance to be applied and the desired release profile, the skilled person may find the optimal swelling degree and a suitable host and guest polymer by performing a number of tests, based on the above teaching.

It has been found, to be very beneficial to provide the guest polymer to have a structure comprising a plurality of beads forming the substantially continuous pathways within the host polymer. By loading the guest monomer into the host polymer using a solvent in its supercritical, near supercritical or dense gas state the resulting guest polymer will form beads like units within the host polymer structure when it precipitates.

The precipitation either occurs during polymerization (if the polymer is not soluble in the polymerization solution) or when the solvent is removed. Advantageously, the supercritical, near super critical or dense gas solvent used comprises $CO_2$ or a combination of $CO_2$ with ethanol, such as up to 5 % by mol. of ethanol. It is believed, that the bead shape is provided when the polymerized monomers reaches a certain size and/or when the polymerized monomers is subjected to a pressure decrease when the pressure of the supercritical, near supercritical or dense gas is released and the supercritical, near supercritical or dense gas solvent used evaporates instantly and leaves the polymerized guest polymer within the holt polymer structure.

In an embodiment the plurality of beads are arranged in side-by-side formations to form the substantially continuous pathways within the host polymer. It has been found, that where the amount of guest polymer is sufficient the beads of guest polymer may form side-by-side formations when the guest is loaded into the host polymer using a solvent in its supercritical, near supercritical or dense gas state.

In an embodiment the host polymer has a structure of a network of strand shaped filaments comprising a plurality of intrastrand pathways and the plurality of beads of guest polymers are arranged along the strand shaped filaments to form substantially continuous pathways within the host polymer. This intrastrand pathways structure may be as described above. It has been found that where silicone is used as host polymer and the residual oils is removed e.g. using a solvent in supercritical, near super critical or dense gas state, the guest polymer may be arranged along the strand shaped filaments and the plurality of beads of guest polymers may adhered to the strand shaped filaments to form the substantially continuous pathways within the host polymer.

In an embodiment the host polymer has a structure of a network of strand shaped filaments comprising a plurality of intrastrand pathways and the guest polymer is provided by soaking the host polymer in a solution of the selected monomers and optional polymerization initiator where the solvent used comprises an organic solvent that has the ability to swell the host polymer and thereby introduce the monomers and polymerization initiator into the network matrix of the host polymer provided by the network of strand shaped filaments. After a selected soaking time the solvent is allowed to evaporate and the polymerisation of the monomers is initiated e.g. by irradiation (e.g. using UV or IR) or by heating e.g. in an oven -e.g. to 70 - 90 °C. After polymerization undesired remaining's of organic solvent may be extracted e.g. using ethanol which is usually an acceptable solvent. The polymerized guest polymer will thereafter be arranged as a surface coating onto the strand shaped filaments of the host polymer.

In an embodiment, the delivery membrane comprises a continuous matrix of the host polymer and a plurality of interconnected paths of the guest polymer, wherein the interconnected paths of the guest polymer preferably extend through the whole thickness of the membrane to thereby provide suitable migration paths for the chemical substance through the delivery membrane.

In an embodiment the delivery membrane comprises a continuous matrix of the host polymer and a plurality of interconnected paths of the guest polymer, the delivery membrane has an outer surface and a plurality of paths of the guest polymer coincide with the inner surface and/or the outer surface. Thereby suitable migration paths for the chemical substance through the delivery membrane are provided.

In order to reduce delay (lag- phase) of release the delivery membrane may comprise one or more incorporated chemical compounds which may be equal to or differ from the chemical substance to be applied in the cavity.

In an embodiment the delivery membrane comprises one or more chemical compounds incorporated in the form of water-soluble particles, such as drugs, buffers, surfactants, fragrances, dyes, flavours, antioxidants, nutrients, hormones, catalysts and/or any combinations thereof. The chemical compound may advantageously be loaded into the delivery membrane by the method described in WO2013/075724.

Advantageously the delivery device is free of organic solvent other than ethanol. This may e.g. be obtained by using the method of loading the guest polymer into the host polymer as described in WO2013/075724.

In an embodiment, the delivery membrane comprises one or more antimicrobial coatings. Such antimicrobial coatings normally prevent or reduce the growth of selected microorganism for a certain time, such as a few days, e.g. 3-5 days. In an embodiment where the delivery membrane has a lag-phase for the substances to migrate through the membrane via the paths of the guest polymer of 1 or more days, e.g. 2-5 days it is particularly desirable that the delivery membrane comprises one or more antimicrobial coatings on its outer side, opposite to its innermost wall surface.

To provide an effective loading of chemical substance into the cavity the delivery device should advantageously have a substantially size e.g. such that the chemical substance may be loaded into the cavity via a lumen formed in the delivery device and/or by using a needle to penetrate the membrane. Advantageously the membrane has a sufficiently elasticity to close such penetration hole after removing the needle.

Advantageously, the inner surface of the delivery membrane has an inner surface area of at least about $0.01cm^2$, such as at least about $0.1 cm^2$, such as at least about $1 cm^2$, such as at least about $2 cm^2$, such as at least about $5 cm^2$, such as at least about $10 cm^2$. Thereby it may be possibly to load the chemical substance using a needle as described above. Where the delivery device is entirely provided by the delivery membrane the delivery membrane needs to have a larger inner surface area, such as at least about $2 cm^2$, such as at least about $5 cm^2$, such as at least about $10 cm^2$.

In an embodiment, the cavity and the inner surface of said delivery membrane is sufficiently large to load drug into said cavity via a loading lumen and/or a needle.

Advantageously the cavity is sufficiently large to load at least 1 ml of fluid into said cavity.

In an embodiment, the cavity is an at least partially collapsed cavity.

In an embodiment the wall comprising the innermost wall surface is inflatable, in other words, the delivery membrane is inflatable or stretchable, e.g. as an inflatable balloon or a section of an inflatable balloon of a balloon catheter.

In an embodiment, the cavity comprises gas, preferably the wall comprising the innermost wall surface comprises a gas escape valve, such as a pressure relief valve to thereby adjust the amount of gas, e.g. when a chemical substance is loaded into the cavity.

Advantageously, the cavity is adapted for being filled and/or refilled with one or more chemical substance in the form of fluids and or particles, such as cells, catalysts, drugs, buffers, surfactants, fragrances, dyes, flavours or any combinations thereof, the cavity is preferably adapted for being filled with one or more chemical substance by injecting via an inlet comprising a valve, such as an inflation lumen (e.g. of a Foley catheter) and/or by injection directly through the delivery membrane.

In an embodiment where the delivery membrane comprises an elastomer host polymer, the chemical substance can advantageously be injected into the cavity via the delivery membrane, and after removing the needle, the elastic properties of the delivery membrane ensure that the penetration hole made by the injection needle immediately contracts and fully closes/heals.

The chemical substance is advantageously in the form of one or more chemical compounds, preferably soluble in water or an aqueous solution, e.g. comprising buffer and other suitable additives.

In an embodiment the cavity comprises chemical substance(s) in the form of one or more chemical compounds in the form of fluids and or particles, such as cells, catalysts, drugs, buffers, surfactants, fragrances, dyes, flavours or any combination thereof.

In an embodiment, the chemical substance comprises a drug, such as an active pharmaceutical ingredient (API). Preferably the drug is selected from antifungal, anticonvulsants, analgesics, antiparkinsons, anti-inflammatories, calcium antagonists, anesthetics, antimicrobials, antimalarials, antiparasitics, antihypertensives, antihistamines, antipyretics, alpha-adrenergic agonists, alpha-blockers, biocides, bactericides, bronchial dilators, beta-adrenergic blocking drugs, contraceptives, cardiovascular drugs, calcium channel inhibitors, depressants, diagnostics, diuretics, electrolytes, enzymes, hypnotics, hormones, hypoglycemics, hyperglycemics, muscle contractants, muscle relaxants, neoplastics, glycoproteins, nucleoproteins, lipoproteins, ophthalmics, psychic energizers, sedatives, steroids sympathomimetics, parasympathomimetics, tranquilizers, urinary tract drugs, vaccines, vaginal drugs, vitamins, collagen, hyaluronic acid, nonsteroidal anti-inflammatory drugs, angiotensin converting enzymes, polynucleotides, polypeptides, polysaccharides, nicotine, adreanal hormones, painkillers, morphine, anticancer and combinations and mixtures thereof.

In an embodiment, the chemical substance comprises drug for treating symptoms of alzheimers, such as T3D-959, donepezil, galantamine, memantine, rivastigmine or combinations thereof. T3D-959 is a dual agonist of the peroxisome proliferator activated nuclear receptor delta/gamma, aka PPAR$\delta$/$\gamma$.

In an embodiment the chemical substance comprises a drug selected from components of the blood clotting cascade, such as factor VIII; birth control drugs, such as levonorgestrel or ethinyl estradiol; anti-virus drugs (antiretroviral), such as anti-HIV drugs (dapivirine, tenofovir) or anti-hepatitis C (ribavirin).

In an embodiment, the chemical substance comprises a compound for human nutrition, such as a vitamin, a protein and/or a mineral or a component comprising one or more of the above-mentioned.

In an embodiment the chemical substance comprises a compound for microbiological nutrition, such as $FeSO_4 \cdot 7H_2O$, $ZnSO_4$, KCI, $MgSO_4, 7H_2O$, $NaNO_3$, Glucose, NaCI, $K_2HPO4$, $NH_4H_2PO4$, $CaC1_2 \cdot 2H_2O$, $FeC1_3 - 6H_2O$, $NaH_2PO4$, $Na_2HPO_4$, $NaHCO_3$, $(NH_4)_2SO_4$ or mixtures comprising any of the mentioned.

In an embodiment, the chemical substance comprises a fragrance compound, a flavor compound and/or a colour compound, e.g. pigment and/or dyes. By applying a colour compound in the chemical substance, the front of the migration of the chemical substance as it migrates through the delivery membrane may be visible.

In an embodiment the chemical substance comprises an active chemical compound and a colour compound, where the colour compound is selected such that its colour depends on the concentration of the active chemical compound, thereby the amount of remaining active chemical compound can be observed externally or internally to the membrane.

In an embodiment the chemical substance comprises a colour compound, where the colour compound is selected such that its can be applied as an indicator for a diagnosis.

Examples of further suitable chemical compound drugs include Anti-cancer drugs, such as Anastrozol, Bendamustine hcl, Bleomycinsulfate, Capecitabine, Cisplatin, Doxirubicinhcl Docetaxeltrihydrate or Gemcitabine hcl, Letrozole; Steroid Hormone drug, such as Dexamethasone, Estradiol, Fluocinolone acetonide, Gestodene, Hydrocortisone, Mifepristone, Mometasone furoate, Progesterone or Prednisolone, Triamcinolone Acetonide; Nootropic and Nutrition drug, such as Aniracetam, Acetyl L-Carnitine, Ademetionine, Disulfate, Tosylate (SAMe), Ademetionine1,4butanedisulfonate, Branched-chain amino acids, (BCAA), CDP Choline, Creatine Monohydrate, DHA EPA EGCG, L-Theanine, 1.3-dimethylamylamine, hcl (DMAA), Oxiracetam, Phenibut, Pramiracetam or Sulbutimiane; Cardiovascular system drug, such as Amlodipine besylate, Alagebrium chloride, Atorvastatin Calcium, Candesartan, Candesartan Cilexetil, Losartan potassium, Phentolamine mesylate, Rosuvastatin calcium or Telmisartan; Antidepressant drug, such as Tianeptine acid, Tianeptine Sodium or Sertraline HCI; Antibiotics and anti-inflammatory drug, such as Amikacin sulphate, Chlorhexidine Collistin, Cyclosporin A, Daptomycin, Gatifloxacin, Gentamycin sulphate, Loxoprofen Sodium, Marbofloxacin, Nitrofurazone, Nitrofurantoin Polymyxin B sulfate, Rapamycin, Rifampicin Rifaximin, Sulphamethizole,Tacrolimus, Trichlosan or Tobramycin (Sulfate); Anti-fungal drug, such as miconazole; Prostaglandin drug, such as Alprostadil (PGE1), Bimatoprost, Dinoprost, trometamol (F2alpha), DL-Cloprostenol sodium, Latanoprost or Misoprostol; Cosmetic peptide drug, such as Argireline Acetate, Acetyl Octapeptide-3, Acetyl, Tetrapeptide-5, Copper peptide /Cu-GHK/AHK, Dipeptide-2 /Pal-tetrapeptide-3, Hexapeptide-9, Hexapeptide-10, L-Carnosine, Matrixyl Acetate, Myristoyl, Pentapeptide-16, Myristoyl Pentapeptide-17, N-Acetyl carnosine, Octapeptide 2(TM-8-NH2), Palmitoyl tripeptide-1(Pal-GHK ) or Palmitoyl Tripeptide-5; Urinary System drug, such as Finasteride, Dutasteride, Hydrochlorothiazide, Mirabegron, Oxbutynin, Spironolactone or Tamsulosin; Other drugs, such as Aprepitant, Benserazide hcl, Citicoline Sodium, Daclatasvir, Dextromethorphan HBr, Donepezil HCI, Entecavir, Fingolimod Hcl, Flavoxate HCL, Formoterol Fumarate, Indocyanine Green, Ipratropium Bromide, Ketanserin tartrate, Ledipavir, Lorcaserin hydrochloride, Lactobionic acid, Levothyroxine sodium, Monoxidil, Obeticholic acid, Phenolphthalein, Pioglitazone HCI,

Pimobendan Pirfenidone, Pregabalin, Sofosbuvir, Tadalafil, Tamoxifen citrate, Tenofovir, disoproxil fumarate, Tiotropium bromide, Tranexamic acid, Ulipristal acetate or Xylometazoline hcl.

In order to have a relatively high release it is desired that the molar mass of the chemical compound of the chemical substance is not too high. In an embodiment the chemical compound has a molar mass of up to about 300,000 g/mol., such as up to about 90.000 g/mol., such as up to about 50.000 g/mol., such as up to about 10.000 g/mol., such as up to about 5000 g/mol.

In an embodiment, the chemical compound comprises particles selected from stem cell(s), catalyst(s), nanoparticle(s) or combinations thereof.

In an embodiment, the cavity comprises particles comprising cells, such as stem cells or microorganisms. The cells are encapsulated in the cavity comprising the delivery membrane and are preferably capable of producing at least one by-product. The by-product is capable of migrating through the delivery membrane, preferably via pathways of the guest polymer when the delivery membrane is in wet condition, i.e. when the guest polymer is in moisture condition.

In an embodiment, the delivery membrane comprises one or more chemical compounds in the form of fluids and or particles, such as cells, catalysts, drugs, buffers, surfactants, fragrances, dyes, flavours or any combinations thereof.

Advantageously, the guest polymer is an aerogel or a xerogel of a hydrogel, preferably obtained by freeze drying the hydrogel or by freeze drying the delivery membrane with hydrogel guest polymer under supercritical or sub-supercritical conditions using a carrier gas such as $CO_2$.

Advantageously, the chemical substance is selected relative to the membrane such that it diffuses through the membrane at zero order kinetics.

In an embodiment, the chemical substance comprises drug trapped in liposomes which are dispersed in a polar liquid, preferably in an aqueous liquid. The liposomes comprise the drug to be released in a solution or dispersion trapped within the respective liposomes. Upon heating the lipid bilayer of the liposome will be ruptured and the drug will be released from the liposome. After being released from the liposome the drug will pass through the delivery membrane to be released from the delivery device with a desired release profile.

The liposome size can vary from very small (0.025 $\mu$m) to large (2.5 $\mu$m) vesicles. Moreover, liposomes may have one or bilayer membranes. On the basis of their size and number of bilayers, liposomes can be classified into one of two categories: (1) multilamellar vesicles (MLV) and (2) unilamellar vesicles. Unilamellar vesicles can also be classified into two categories: (1) large unilamellar vesicles (LUV) and (2) small unilamellar vesicles (SUV) [16]. In unilamellar liposomes, the vesicle has a single phospholipid bilayer sphere enclosing a drug in solution or dispersion. In multilamellar liposomes, vesicles have an onion structure and several unilamellar vesicles may form on the inside of the other with smaller size, making a multilamellar structure of concentric phospholipid spheres separated by layers of drug in solution/dispersion.

In an embodiment the chemical substance comprises drug trapped in liposomes and the rupturing of the lipid bilayer of the liposome is provided by heating applied by irradiation, light or conventional heat.

In an embodiment, the chemical substance comprises drug trapped in liposomes together with metal particles, preferably gold particles and the and the rupturing of the lipid bilayer of the liposome is provided by irradiating - e.g. by laser light or IR light, the metal particle thereby heating the particles and liposomes close to a heated metal particle will have its lipid bilayer ruptures such that the trapped drug is released from the liposome.

In an embodiment, the chemical substance comprises drug trapped in liposomes together with metal particles, preferably gold particles, and together with light emitting units which light emitting units can be triggered to emit light to heat the metal particles. The rupturing of the lipid bilayer of the liposome is provided by triggering the light emitting unit thereby heating the particles and liposomes close to a heated metal particle will have its lipid bilayer ruptures such that the trapped drug is released from the liposome.

The light emitting units may e.g. be quantum dots often referred to as Q-dots. Examples of quantum dots are described in US 7498177 and the quantum dots available from Life Technologies Europe BV. include more than 150 different product configurations with emission wavelength spanning in a broad wavelength range for examples quantum dots with the respective emission wavelengths: 525, 545, 565, 585, 605, 625, 655 and IR 705 and 800 nm. The quantum dots may be excited by irradiating from outside of the delivery device e.g. by laser light or IR light.

In an embodiment, the light emitting units are light emitting diodes, such as the micro light-emitting diodes ($\mu$-ILED array) described in "Wireless Optofluidic Systems for Programmable In Vivo Pharmacology and Optogenetic" by Jae-Woong Jeong et al. Cell 162, 662-674, July 30, 2015 Elsevier Inc. DOI: http://dx.doi.org/10.1016/j.cell.2015.06.058

As described herein the micro light-emitting diodes may be triggered and even programmed wireless.

In an embodiment, the chemical substance comprises drug trapped in liposomes together with micro light-emitting diodes as described above - i.e., without metal particles and the micro light-emitting diodes is triggered or even programmed by wireless connection.,

In an embodiment, the delivery device comprises a coating on at least a part of its inner or outer surface. In an embodiment also another part or parts of the delivery device comprise(s) a coating. In an embodiment the coating is selected from an adhesive, such as silicone adhesive, pressure sensitive adhesive, PU adhesive or TPU skin adhesive e.g. as marketed by MaterialScience. Baymedix. In an embodiment, the coating is a biopolymer, such as Hyaluronic acid (HA), chitosan, lignin, alginate, pectin or combinations thereof. In an embodiment the coating is a hydrophilic coating e.g. a hydrogel coating e.g. for reducing friction against skin or mucosa. Preferably at least a part the inner surface and/or the outer surface of the delivery membrane comprises a coating.

In an embodiment, the coating is a noble metal coating (i.e. bactiguard coating) or other drug delivering coatings, nitrofurazone, antibiotic silver, etc.

In an embodiment the coating is a hydrophobic coating (e.g. silicone oil) - e.g. for reducing friction from insertion.

In an embodiment, the delivery device comprises a coating on at least a part of its inner or outer surface where the coating is made of a material substantially identical to the guest polymer.

In an embodiment, the delivery device is a balloon catheter comprising at least one balloon forming the cavity and advantageously the delivery membrane is a part of or the entire balloon of the balloon catheter.

In an embodiment the delivery device is a balloon catheter as described in US 6602243 with the difference that at least a section of the wall of the balloon is a delivery membrane as described herein.

Advantageously the delivery device is a urinary catheter, such as a Foley catheter and the delivery membrane is the balloon or a section thereof, the balloon is adapted to being inflated using an inflation liquid comprising the chemical compound(s) of the chemical substance. After the inflation liquid comprising the chemical compound(s) has been used as inflating liquid the chemical compound(s) will migrate through the delivery membrane.

Patients using urinary catheters are very often infected with bacteria which often can be very uncomfortable or even painful to the patients and which may impair the perceived quality of life of the patient. By using the delivery device catheter of the invention and a drug against the bacteria as a chemical compound in the inflation liquid, the infection may be avoided or treated during use of the catheter.

In an embodiment, the balloon is a compliant balloon which continuously expands upon filling fluid into the balloon e.g. upon increasing the pressure within the balloon.

In an embodiment, the balloon is a non-compliant balloon which expands until a specific size or size range is reached upon filling fluid into the balloon, e.g. upon increasing the pressure within the balloon. Such type of balloon is for example described in WO 2010/042869. In an embodiment the delivery device of the invention is a catheter as described in WO 2010/042869 with the difference that at least a section of the balloon wall is a delivery membrane as described herein.

In an embodiment, the balloon is a folded balloon which unfolds upon filling fluid into the balloon.

In an embodiment, the catheter comprises a diagnostic probe, such a probe comprising a marker such as a fluorescence marker. The probe is adapted for detection of at least one biomarker such as, but not limited to, pH value, temperature, moisture level or infection level and combinations thereof, e.g. by multiplexing technique.

In an embodiment, the diagnostic probe is configured for changing upon a change of the at least one biomarker. The change of the probe is preferably optically readable. The change may for example be a change of colour. Suitable probes can be found in "The Molecular Probes® Handbook-A Guide to Fluorescent Probes and Labelling Technologies", freely available on the Internet:

https://www.lifetechnologies.com/dk/en/home/references/molecular-probes-the-handbook.html

In an embodiment, the probe is incorporated or mounted in a distal end of the catheter, such as in a distal tip of the catheter, such that it will be positioned in the bladder of a patient during use. If an infection is developing the infection can be detected by the probe. The probe can for example be read out optically or electrically e.g. using an electrochemical reader. For electrochemical read-out the catheter may comprise one or more incorporated electrical wires or strips such that the electrical potential of the probe can be read out. This may be in the form of impedance read-out.

In an embodiment, the probe can be observed externally or internally to the membrane e.g. visually or optically e.g. where the probe comprises a fluorescent marker and/or an enzymatic marker.

In an embodiment, the catheter comprises a read-out structure for reading the probe and transmitting the read signal to a displaying element for visually or audibly displaying, wherein the read-out structure comprises an optical or electrochemical reader.

A read-out structure e.g. based on optical read-out of for example a fluorescence based signal or based on electrochemical read-out, will advantageously report the infection level and denote the type of microorganism. Depending on the type of microorganism one or more drugs can be introduced in the inflation liquid for the balloon for local treatment of the infection.

In an embodiment, the catheter comprises an optical read-out structure. The catheter comprises a drainage tube and a distal tip. The drainage tube comprises a channel for draining urine. The channel extends to the tip, and the tip comprises or carries the probe. The channel is configured for functioning as a waveguide, e.g. when filled with liquid, such as urine or water. The probe is advantageously positioned such that at least a part of a light beam fed to the channel reaches the probe, such that reflected or scattered light can be detected by an optical reader. Thereby the channel forms a light beam path for the detection.

In an embodiment, the catheter comprises an optical read-out structure. The catheter comprises an inflation tube and a distal tip. The inflation tube comprises a channel for inflating the balloon. The channel extends to the tip, and the tip comprises or carries the probe. The channel is configured for functioning as a waveguide e.g. when filled with liquid, such as buffer or water. The probe is advantageously positioned such that at least a part of a light beam fed to the channel reaches the probe, such that reflected or scattered light can be detected by an optical reader. Thereby the channel forms a light beam path for the detection.

In one embodiment, the inflation channel guides light to heat the balloon via gold particles or other heating mechanisms to increase the release of drug. The guiding of light for heating the balloon may be coupled to the sensor in a feed-back loop.

In an embodiment, the catheter comprises an optical read-out structure, wherein the catheter comprises a drainage tube and/or an inflating tube and a distal tip. The drainage tube comprises a channel for draining urine, and the inflation comprises a channel for inflating the balloon. The at least one of the channels extends to the tip and the probe is fixed on an outer surface of the tube, wherein the tube has a transparent window to the probe. The at least one channel is configured for functioning as a waveguide e.g. when filled with liquid, such as urine or water for reading scattered light from the probe. In an embodiment, the probe is mounted on the balloon and the inflation lumen is applied as an optical waveguide.

In an embodiment, the delivery device is a ring-shaped delivery device and the cavity is ring-shaped or semi-ring-shaped within the ring-shaped delivery device. The ring shaped delivery device may in principle be used for delivery of any type of drug.

In an embodiment, the delivery device is a vaginal ring preferably adapted to be positioned to surround the cervix of a female mammal, such as a human or mammal, such as a human or an animal e.g. a horse, a dog or a cat.

In an embodiment the delivery device is a contraceptive delivery device such as a vaginal ring (P-ring) containing a drug for preventing pregnancy optionally in combination with one or more other drugs, such as in the form of a drug combination for preventing HIV and/or pregnancy.

In an embodiment, the delivery device is a contraceptive delivery device for a mammal, such as a human or a cat.

Such contraceptive delivery device for a cat may for example be used in replacement of spaying or neutering the cat, thus the contraceptive delivery device is much gentler to the cat and complications which are often linked to spaying or neutering by surgery may be avoided.

In an embodiment, the delivery device contains in its cavity a drug for treating symptoms of alzheimers, such as T3D-959, donepezil, galantamine, memantine, rivastigmine or any combinations thereof.

In an embodiment, the delivery device contains in its cavity a substance comprising drug(s) selected from Abomorphin, Hormone replacement, Prograstrorel, cyproterone and combinations thereof.

In an embodiment, the delivery device is a contraceptive device e.g. shaped as a ring and the cavity is formed as a ring or a semi-ring within the device. Advantageously the delivery device is a contraceptive device shaped as a hollow ring. In an embodiment, the hollow ring consists entirely of the delivery membrane forming the cavity into which the chemical substance can be injected e.g. using an injection needle as described above. In an embodiment the entire hollow ring is formed by the delivery membrane and the chemical substance, where the delivery membrane forms the cavity comprising the chemical substance in dry or liquid e.g. dissolved or partly dissolved form.

In an embodiment, the delivery device is a P-ring (vaginal ring) containing a drug combination for preventing HIV or other infections and/or pregnancy. Advantageously, the P-ring is not expandable but retains its shape during use. In an embodiment, the P-ring cavity comprises a hydrogel forming a reservoir for the substance to be delivered by the delivery device in form of a P-ring.

In an embodiment, the delivery device is a capsule, such as a capsule comprising the delivery membrane which is defining the cavity, and where the chemical substance is or is to be injected into the cavity. In an embodiment, the delivery device is a capsule having a volume of at least about $0.01 cm^3$, such as a volume of at least about $0.1 cm^3$.

Advantageously, the capsule having a volume of at least about $0.05 cm^3$, such as a volume of at least about $0.1 cm^3$, such as a volume of at least about $0.5 cm^3$, such as a volume of at least about $1 cm^3$, such as a volume of at least about $2 cm^3$, such as a volume of at least about $5 cm^3$. Thereby the capsule may be loaded with chemical substance using a needle.

In an embodiment, the capsule is shaped as a sphere. Alternatively the capsule may have other shapes, such as oval or egg-shaped.

In an embodiment, the capsule is oblong, egg shaped, oval and/or flattened. The term "flattened" means that the capsule has a shape with two opposite substantially flat outer surfaces, which are is substantially parallel planes.

Advantageously the capsule has a smallest diameter of at least about 5 mm, such as at least about 1 cm, such as at least about 2 cm.

In an embodiment, the capsule comprises drug trapped in liposomes which are dispersed in a polar liquid as described above. Such capsule may e.g. be implanted into a mammal for controlled drug release. The drug trapped in liposomes which are dispersed in a polar liquid may be replaced at preselected intervals or when needed. The drug may e.g. be a pain killer which the patient can trigger to be released.

In an embodiment, the drug is Rivastigmine (sold under the trade name Exelon) which is a parasympathomimetic or cholinergic agent for the treatment of symptoms of mild to moderate dementia of the Alzheimer's type and dementia due to Parkinson's disease. The drug can be administered orally or via a transdermal patch; the latter form reduces the prevalence of side effects which typically include nausea and vomiting. The drug is eliminated through the urine, and appears to have relatively few drug-drug interactions.

In an embodiment, the capsule is adapted for use in waste water treatment or in fuel-cells.

The invention also relates to a delivery device suitable for delivering a chemical substance comprising one or more chemical compounds, the delivery device comprises a closed cavity comprising the chemical substance, the cavity is defined by an innermost wall surface, wherein at least a section of the inner wall surface constitutes an inner surface of a delivery membrane wherein the delivery membrane comprises an interpenetrating polymer network substrate comprising a host polymer and a guest polymer, where the guest polymer is interpenetrating the host polymer to form substantially continuous pathways within the host polymer.

Preferably, the delivery device is as described above wherein the chemical substance is contained in the closed cavity.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

All features of the invention including ranges and preferred ranges can be combined in various ways within the scope of the invention, unless there are specific reasons for not combining such features.

## BRIEF DESCRIPTION OF DRAWINGS AND EXAMPLE

The invention will be explained in more detail below in connection with a preferred embodiment and with reference to the drawings in which:

FIG. 1 is a chart of the mass in mg that migrates through the balloons over time in days of the IPN sample and the blind prior art sample of example 1.

FIG. 2 shows an embodiment of a delivery device of the invention in the form of a balloon catheter.

FIG. 3 is a schematic diagram of use showing an embodiment of a balloon catheter of the invention.

FIG. 4 is a schematic drawing of the human female reproductive system comprising the uterus and with an embodiment of the p-ring of the invention mounted to surround the cervix.

FIG. 5 is a schematic side view of the human female reproduction system corresponding to the drawing of fig.4.

FIG. 6 illustrates a capsule of an embodiment of the invention comprising a chemical substance.

FIG. 7a is a cryo-SEM image of a silicone host polymer.

FIG. 7b is a corresponding illustration of the silicone host polymer of FIG. 7a.

FIG. 8a is a cryo-SEM image of the silicone host polymer with interpenetrating guest polymer with a structure comprising a plurality of beads.

FIG. 8b is a corresponding illustration of the silicone host polymer/guest polymer IPN of FIG.8.

FIG. 9a is a SEM image of the silicone host/guest polymer IPN of FIGs 8a and 8b which has been swollen with water.

FIG. 9b is a corresponding illustration of water swollen the silicone host polymer/guest polymer IPN of FIG. 9a.

FIG. 10 comprises the illustrations of FIGs 7b, 8b, 9b side by side.

FIG 11 is a SEM image of a silicone host polymer before being subjected to residual extraction.

FIG 12 is a SEM image of the dry silicone host/guest polymer IPN of Fig 9a.

FIG. 13 illustrates a capsule of an embodiment of the invention comprising a chemical substance comprising liposomes.

The figures are schematic and may be simplified for clarity. Throughout, the same reference numerals are used for identical or corresponding parts.

### Example 1

*Diffusion of Methylene Blue in Foley catheters*

Purpose: An experiment was designed to qualitatively and quantitatively measure the diffusivity of methylene blue through the retention balloon of a urinary Foley catheter and to calculate the diffusion coefficient, *D*, for a catheter according to an embodiment of the invention and a prior art catheter.

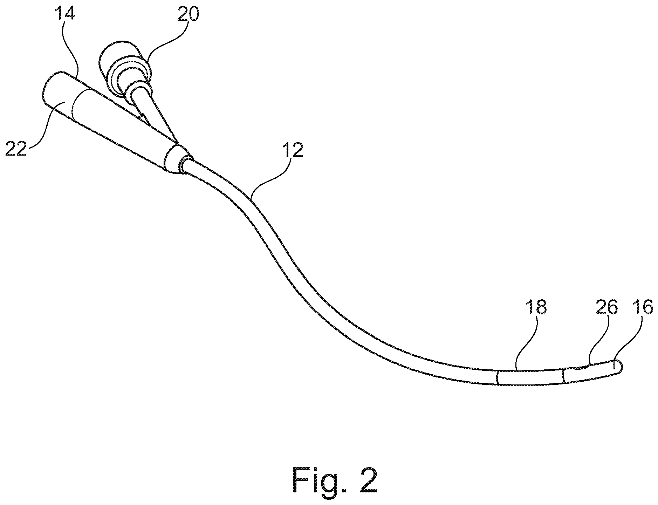

Materials and Methods: Two catheters were obtained from Bactiguard. One of the catheters was treated by removing the balloon and replacing it with an IPN of silicone as host polymer and hydrogel as guest polymer where the hydrogel content was 24% (called the IPN catheter). The other catheter was untreated (called the prior art catheter). The IPN catheter and the prior art catheter were inflated with aqueous glycol solution to check for any leaking. After rinsing and cleaning them, they were each filled with 10 ml 10 mg/mL aqueous methylene blue solution and each catheter was partly (including the tip (16), hole for drainage (26), balloon (18) and 2/3 of the shaft (12) on Fig.2) submerged into 1 L distilled water with stirring at 200 rpm at ambient temperature. The volumes were kept constant during the experiment. The concentration of methylene blue in the 1 L surrounding water was periodically measured by applying a Thermo Scientific Evolution 220 UV-vis spectrophotometer at 664 nm.

Results: The experiment showed that methylene blue migrates through the balloon material on the IPN catheter turning the surrounding liquid blue. Over the period of the experiment (24 days) it was not possible to detect methylene blue in the surrounding liquid of the prior art catheter. This strongly indicates that methylene blue cannot diffuse through the prior art balloon, but the IPN treatment (current invention) enables diffusion.

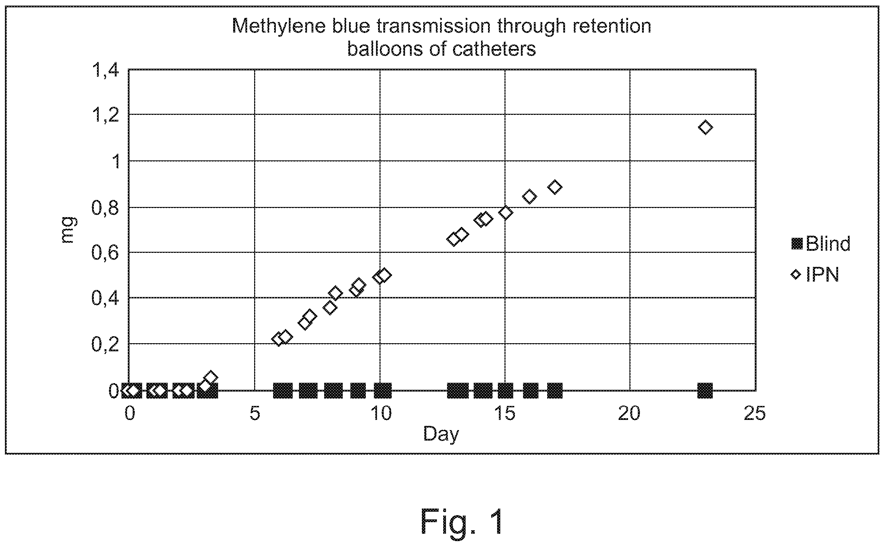

Figure 1 is a chart of the mass in mg that diffuses through the balloons over time in days of the IPN catheter and the blind prior art catheter (blind sample).

The blind sample showed no diffusion into the solvent whereas the IPN catheter had a lag-phase of 3 days before methylene blue could be detected in the solvent.

The diffusion coefficients, *D*, of methylene blue in IPN catheter and blind sample can be estimated by the equation (1).

$$D = \frac{Q \cdot X}{\Delta c \cdot A \cdot t} \qquad (1)$$

Where *Q* is the total amount of methylene blue (permeant) that has passed through an area, *A*, during time, *t*. *X* is the thickness of the balloon and $\Delta c$ is the difference in concentration between the outer surface and the inner surface of the balloon.

*Q* can be obtained by determining the linear regression for the IPN after the lag-phase has ended i.e. from day 3 to day 14 on figure 1. The linear regression is given by equation (2)

$$m = 0.061 \cdot t - 0.1236 \qquad (2)$$

*Q* is the slope of the linear regression, i.e. 0.061 mg per day.

It is not possible to measure the thickness of the balloon, *X*, when the balloon is inflated. However, *X* can be estimated by assuming that the volume of the balloon material does not change when the balloon is inflated. In the inflated condition the shape of the balloon can be approximated by a spherical shell and in the un-inflated (relaxed) condition the shape of the balloon can be approximated with a hollow cylinder. The volume of the balloon material can be calculated from the relaxed condition and can then be used to calculate the thickness of the balloon in the inflated condition.

The volume of a hollow cylinder, $V_{hc}$, is given by equation (3).

$$V_{hc} = h \cdot \pi \cdot (r_1^2 - r_2^2) \qquad (3)$$

Where *h* (2.50 cm) is the height of the cylinder and $r_1$ (0.365 cm) and $r_2$ (0.315 cm) is the outer and inner radii respectively. This yields a total volume of the balloon material of $V_{hc}$ = 0.2670 cm$^3$.

$$V_{hc} = 2.50\,\text{cm} \cdot \pi \cdot ((0.365\,\text{cm})^2 - (0.315\,\text{cm})^2) = 0.2670\,\text{cm}^3 \qquad (4)$$

As the cavity between the shaft and the balloon is filled with the inflation liquid, the balloon material will assume the shape of a spherical shell. However, the volume of the balloon material will still be $V_{ss} = V_{hc} = 0.2670$ cm$^3$. The volume of a spherical shell, $V_{ss}$, is given by equation (5).

$$V_{ss} = \frac{4}{3} \cdot \pi \cdot (r_1^3 - r_2^3) \tag{5}$$

The thickness of the balloon material in the inflated state, $X$, is given by equation (6).

$$X = r_1 - r_2 \tag{6}$$

$r_1$ can be measured to 1.25 cm. The inner radius, $r_2$, can be calculated by isolating $r_2$ in equation (5). This is given by equation (7).

$$r_2 = \sqrt[3]{r_1^3 - \frac{3 \cdot V_{ss}}{4 \cdot \pi}} = \sqrt[3]{(1.25 \,\text{cm})^3 - \frac{3 \cdot 0.2670 \,\text{cm}^3}{4 \cdot \pi}} = 1.2362 \,\text{cm} \tag{7}$$

$X$ can now be calculated from equation 6. This is given by equation (8).

$$X = 1.25 \,\text{cm} - 1.2362 \,\text{cm} = 0.0138 \,\text{cm} \tag{8}$$

Now, we have values for the parameters in the numerator in equation (1) and we need to find values for the denominator.

$\Delta c$ is the concentration difference between the outer surface and the inner surface of the balloon material. As the initial concentration of methylene blue is 10 mg/ml and the cavity between the shaft and the balloon material is filled with 10 ml, there is 100 mg methylene blue in the cavity. As can be seen on figure 1 about 1 mg methylene blue is migrated though the balloon material over a period of 20 days. This is only 1%. It is therefore safe to assume that the concentration at the inner surface is 10 mg/ml throughout the experiment. As there is constant mixing on the outside of the catheter the concentration can be assumed to be homogenously distributed. Again, 1 mg methylene blue in 1 L = 1000 ml water is 0.001 mg/ml ≈ 0 mg/ml. Therefore $\Delta c$ is about 10 mg/ml throughout the experiment.

A is the surface area of the balloon material in the inflated condition. The area of a sphere with radius r = 1.25 cm is given by equation (9).

$$A = 4 \cdot \pi \cdot r^2 = 4 \cdot \pi \cdot (1.25 \,\text{cm})^2 = 19.63 \,\text{cm}^2 \tag{9}$$

now, we can estimate the diffusion coefficient of methylene blue though the balloon material by inserting in equation (1). This is given by equation (10).

$$D = \frac{Q \cdot X}{\Delta c \cdot A \cdot t} = \frac{0.061 \,\text{mg} \cdot 0.0138 \,\text{cm}}{10 \,\text{mg/cm}^3 \cdot 19.63 \,\text{cm}^2 \cdot 86400 \,\text{s}} = 4.95 \cdot 10^{-11} \,\text{cm}^2/\text{s} \tag{10}$$

As it was not possible to detect any methylene blue in the surrounding liquid of the prior art catheter throughout the experiment the diffusion coefficient is $D = 0$ cm²/s.

Conclusions: Methylene blue diffused from the cavity through the balloon material on the IPN catheter into the solvent, while no diffusion with the prior art catheter was observed. The diffusion coefficient for the delivery membrane of the IPN catheter was calculated to be $4.95 \cdot 10^{-11}$ cm²/s.

The balloon catheter shown in FIG. 2 is of the Foley type and comprises a catheter body 12 with a proximal end 14 and a distal end 16. The catheter also includes a balloon 18, an inflation lumen 20, a drainage lumen 22, and an adapter 24.

The balloon 18 is deflated for insertion into a patient. The balloon 18 is disposed near the distal end 16. The inflation lumen 20 extends within the catheter body 12 from the proximal end 14 to the balloon 18, in fluid communication with the balloon 18, for inflating and deflating the balloon 18.

The catheter drainage lumen 22 extends from the proximal end 14 to the distal end 16. The distal end 16 includes an opening 26 in fluid communication with the drainage lumen 22 to facilitate drainage of urine from the bladder of a patient.

In the shown embodiment the balloon 18 is the delivery membrane, which can be inflated e.g. with a fluid comprising chemical substances for being delivered through the delivery membrane as discussed above.

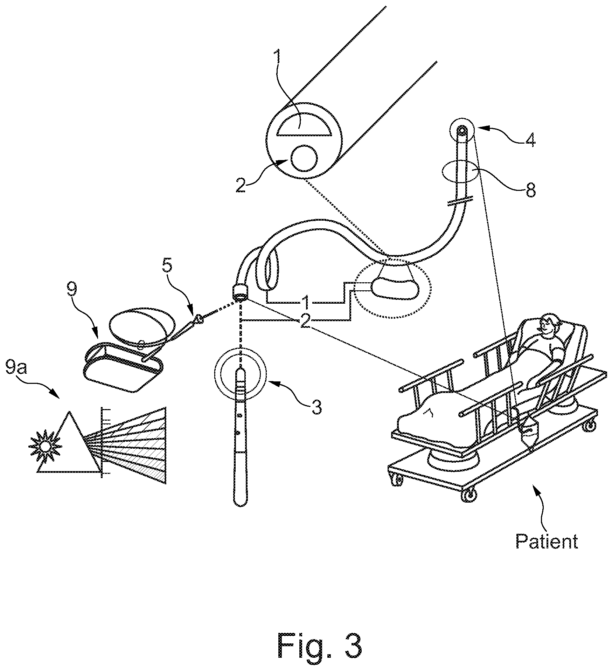

FIG. 3 is a schematic diagram of use another embodiment of a balloon catheter of the invention.

The balloon catheter shown in FIG. 3 comprises a catheter body 12 with a proximal end and a distal end with a probe 4. The catheter also includes a balloon 8 expanded with a fluid comprising chemical substances to be delivered through the balloon wall which constitutes the delivery membrane. The catheter further comprises an inflation lumen 2, a drainage lumen 1. A cut-out section of the body illustrates that the inflation lumen 2 and the drainage lumen 1 extend along the body.

As illustrated in FIG. 3 the balloon catheter is used by a patient.

The probe 4 is advantageously as described above, e.g. a probe with an optical biomarker.

The inflation lumen is configured for functioning as a waveguide e.g. when filled with liquid. For reading out a laser 3 is arranged to transmit a beam via the inflation lumen. The probe 4 is positioned such that at least a part of a light beam fed to the channel (inflation lumen) 2 reaches the probe 4, such that reflected or scattered light can be detected by an optical reader 9. And a readout spectrum can be obtained as illustrated with the spectrum 9a. Thereby the channel forms a light beam path for the detection.

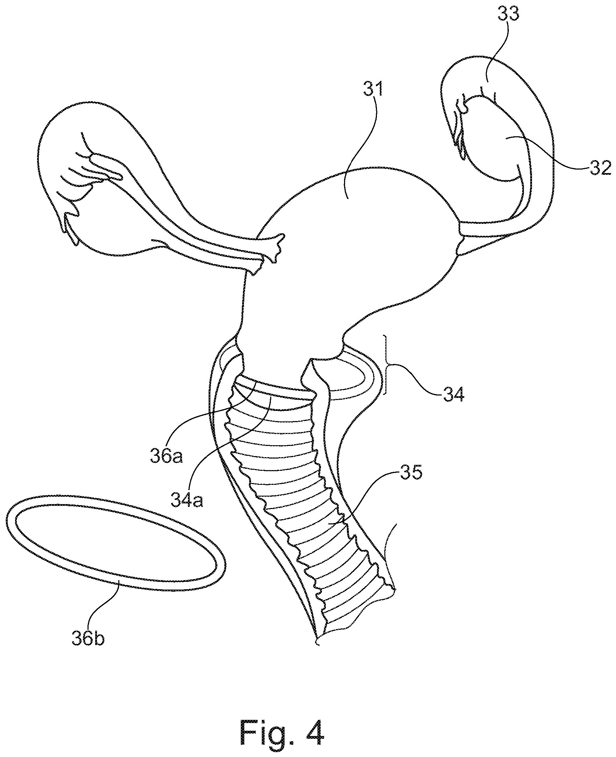

The human female reproductive system shown in FIG. 4 comprises the uterus 31, the fallopian tubes 32, the ovaries 33 and the cervix 34. The lower part of the cervix 34a leads into the vagina 35 and the P-ring 36a is mounted in the vagina to surround the lower part of the cervix 34a. Reference 36b illustrated the p-ring prior to mounting.

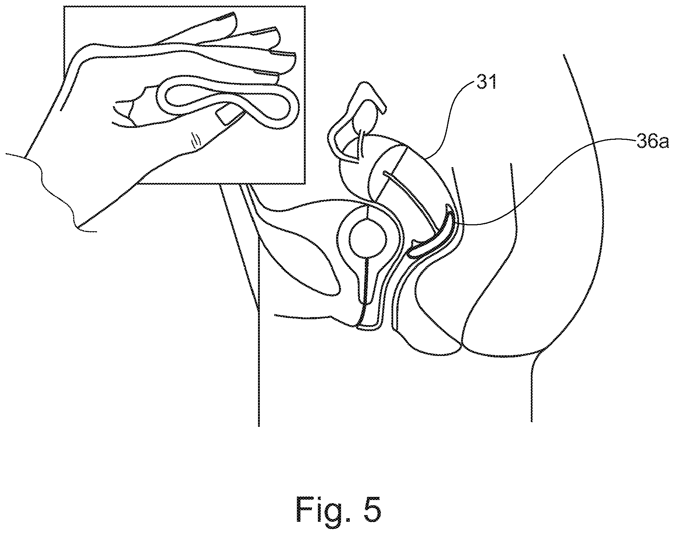

FIG. 5 is a schematic side view of the human female reproduction system corresponding to the drawing of FIG.4. It is illustrated that the P-ring is very flexible and is easily bendable by hand.

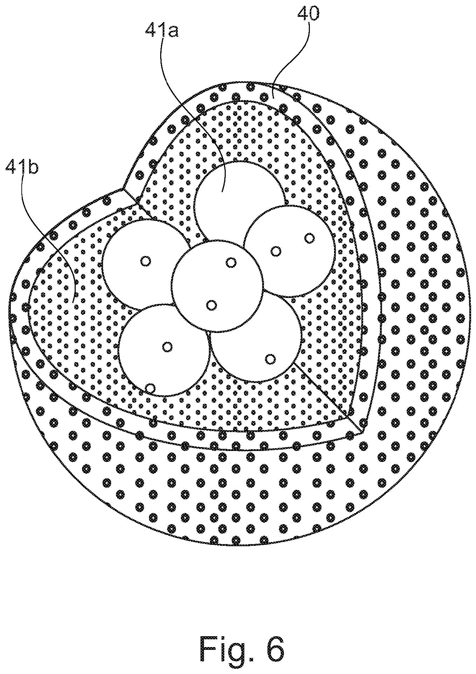

The capsule shown in figure 6 comprises a capsule wall 40 constituting the delivery membrane and forming a cavity comprising the chemical substances 41a in a fluid 41. In the shown embodiment a section of the capsule is cut out for illustrative purposes. In an alternative embodiment the whole cavity encapsulated by the capsule wall 40 is filled with a chemical substance in dry form.

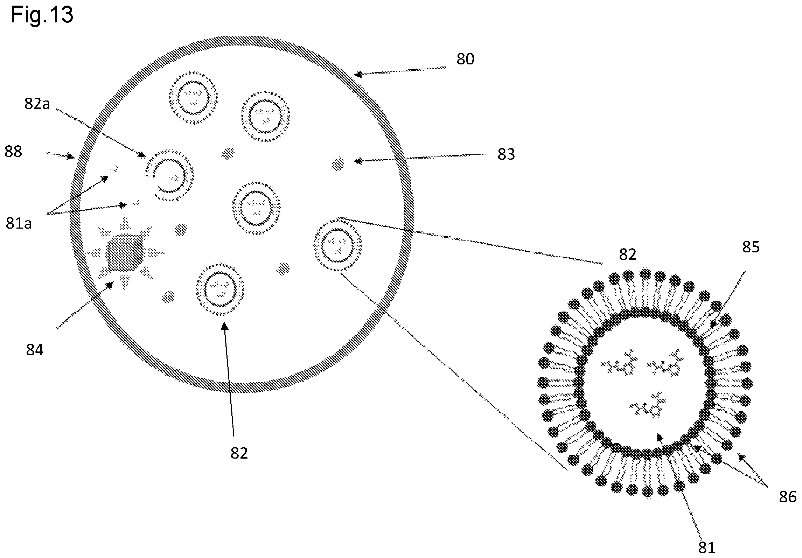

The capsule of FIG. 13 comprises a capsule wall 80 constituting the delivery membrane and forming a cavity comprising the chemical substances. The chemical substance comprises drug 81 trapped in liposomes 82 together with gold nanoparticles 83, and one or more light emitting units 84, such as quantum dot(s) or micro light-emitting diode(s).

One of the Liposomes is illustrated in enlarged view and here it can be seen that the liposome 82 comprises a lipid bilayer 85 with polar ends 86 turning inwards and outwards.

The drug 81, 81a is preferably Rivastigmine.

The light emitting unit 84 may advantageously be triggered from external impact e.g. as described above. When the light emitting unit 84 emits light it will be absorbed by the gold particles 83, and the temperature of the gold particles 83 will increasing. When the liposomes reaches a certain temperature they rupture as shown with the ruptured liposome 81a and the drug will be released from the liposome. The released drug may now freely migrate across the delivery membrane 80 membrane to the target spot for the drug, e.g. if implanted in the brain to the exposed brain tissue. Rivastigmine can thereby be released to the brain tissue with a desired release profile. When the amount of liposomes are reduced or there is no more left the substance within the capsule 88 may be withdrawn using a needle and it may be replaced with fresh substance.

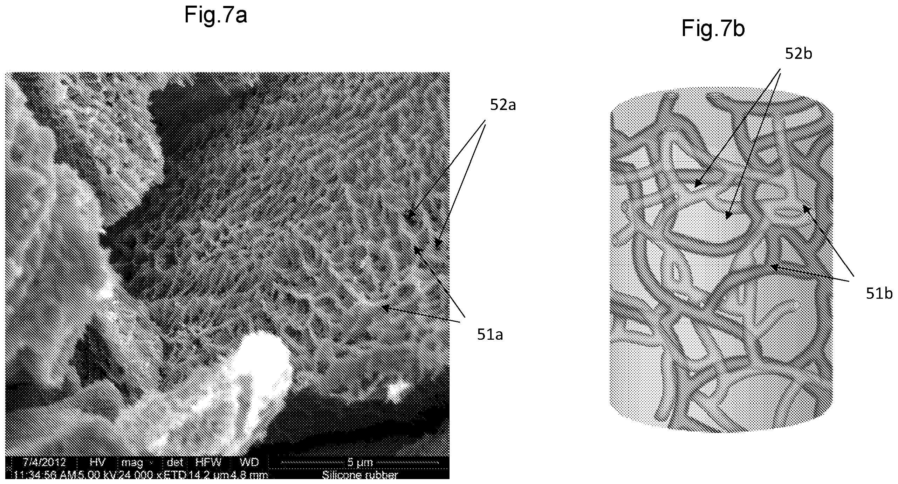

The silicone host polymer shown in FIG. 7a is a PDMS silicone polymer cooled in liquid nitrogen (T ~ -196°C) and removed from the nitrogen immediately prior to acquiring the cryo-sem image. The morphology/structure of PDMS have been frozen by placing the sample in liquid nitrogen, which has a temperature below the glass transition temperature of silicone (Tg = -125°C). It can be seen that the host polymer has a structure of a network of strand shaped filaments 51a and comprises a plurality of intrastrand pathways 52a. The strand shaped filaments are substantially flat.

In Fig. 7b the same host polymer is illustrated with the network of strand shaped filaments 51b and the intrastrand pathways 52b.

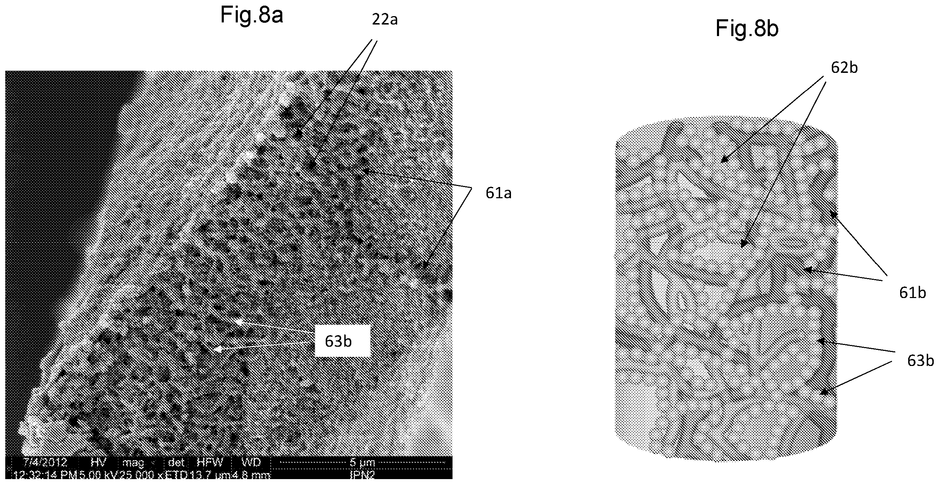

FIG. 8a show the same silicone host polymer with interpenetrating guest polymer also in liquid nitrogen (T ~ -196°C) with a structure comprising a plurality of beads 63a forming a substantially continuous pathways within the host polymer. The guest polymer has been loaded into the host polymer using $CO_2$ in dense gas state as solvent. It can be seen that the plurality of beads 63a of guest polymers are arranged along the strand shaped filaments 61a to form the substantially continuous pathways within the host polymer. It can be seen that the intrastrand pathways 62a are only partly filled. It is believed that this has the effect of making the final IPM delivery membrane very pliable and soft.

It can be seen that the guest polymer has affinity to the host polymer.

Fig. 8b illustrates the silicone host polymer with interpenetrating guest polymer of FIG. 8a and it can be seen more clearly that the beads 63b of guest polymers are arranged along the strand shaped filaments 61b to form the substantially continuous pathways within the host polymer and that the intrastrand pathways 62b are only partly filled.

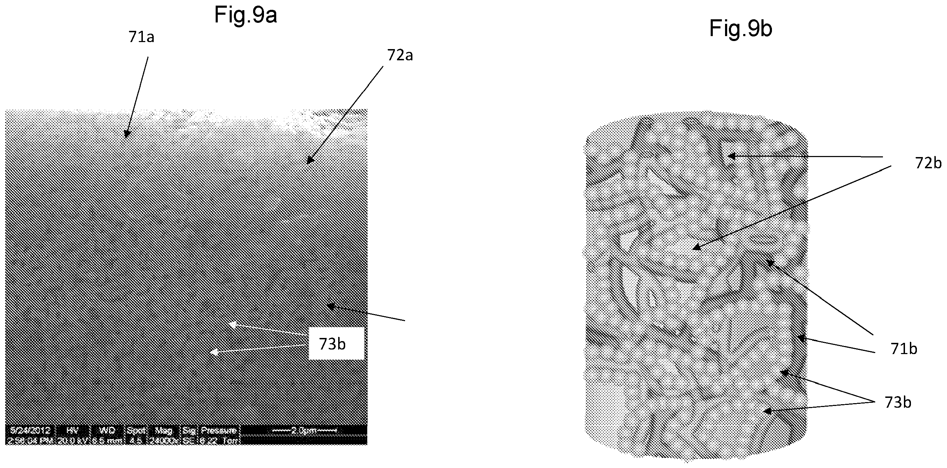

In FIG. 9a the silicone host/guest polymer IPN of FIGs 8a and 8b which has been swollen with water at room temperature. The strand shaped filaments 71a are almost fully covered with the beads 73a which have swollen and thereby increased in size. In the SEM image the intrastrand pathways 72a can only slightly be seen. In the corresponding illustration in FIG. 9b it can be seen that the guest polymer beads 73b has been swollen. For illustrative purpose the swollen guest polymer beads are illustrated in less swollen state than in the real life cry-SEM image of FIG. 9a. However, it can be seen that the swollen guest polymer beads takes up more of the intrastrand pathways 72b between the strand shaped filaments 71b.

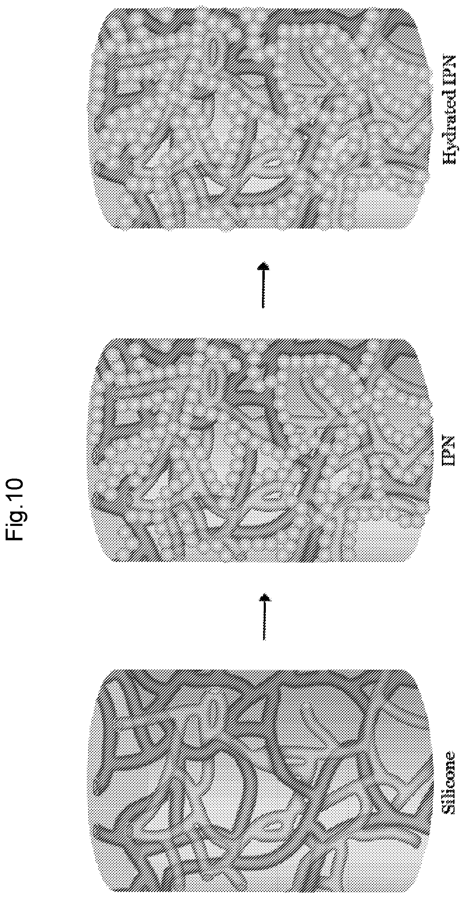

In fig 10 the illustrations of FIGs 7b, 8b, and 9b are shown side by side and it can be seen how the host alone differs from the silicone host/guest polymer IPN and further the changes when the silicone host/guest polymer IPN is swollen with water.

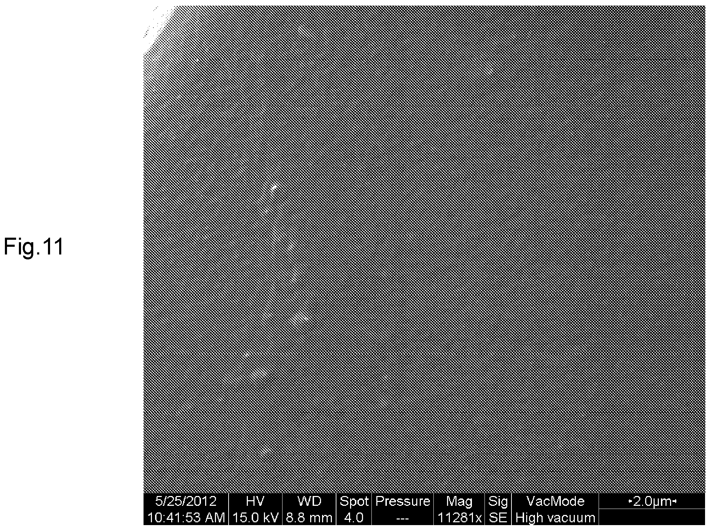

FIG 11 shows a SEM picture the PDMS silicone polymer at room temperature. There is no visible network of strand shaped filaments and intrastrand pathways, because the temperature is much higher than the glass transition temperature of silicone (Tg = - 125°C).

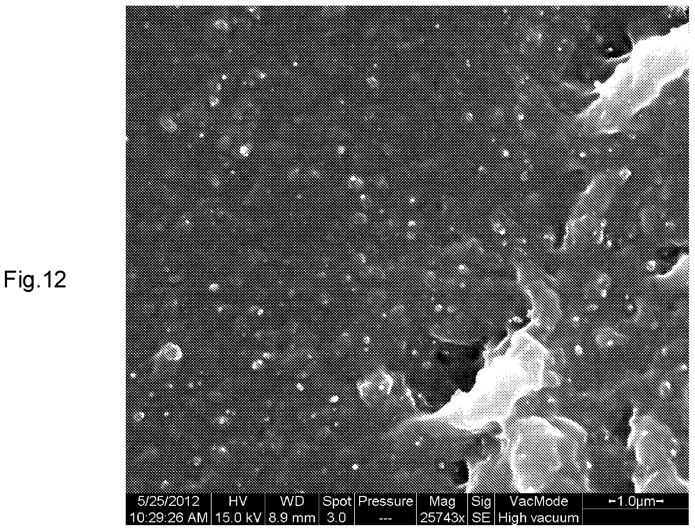

In fig 12 a SEM image of the dry silicone host/guest polymer IPN at room temperature of Fig 9a is shown and it can be seen that the surface as a structure comprising a plurality of bead like structures. The guest polymer (hydrogel) has a Tg of 100°C and is solid at room temperature. Therefore the hydrogel takes the shape as solid beads. The host polymer (silicone) has a Tg of -125°C and therefore behaves as a liquid at room temperature and embeds the solid hydrogel beads.

Although embodiments have been described and shown in detail, the invention is not restricted to these, but may also be embodied in other ways within the scope of the subject matter defined in the following claims. In particular, it is to be understood that other embodiments may be utilized and structural and functional modifications may be made without departing from the scope of the present invention.

## Claims

1. A delivery device suitable for delivering a chemical substance comprising one or more chemical compounds, the delivery device comprises a closed cavity, the cavity is defined by an innermost wall surface, wherein at least a section of the inner wall surface constitutes an inner surface of a delivery membrane wherein the delivery membrane comprises an interpenetrating polymer network substrate comprising a host polymer and a guest polymer, where the guest polymer is interpenetrating the host polymer to form continuous pathways within said host polymer, preferably said delivery membrane has an outer surface opposite to said inner surface of said delivery membrane, said continuous pathways of said guest polymer within said host polymer extend from the inner surface to the outer surface of said delivery membrane.

2. The delivery device of claim 1, wherein the host polymer is a cross-linked polymer such as a cross-linked elastomer, e.g. thermoplastic elastomer (TPE), polyolefin elastomer (POE), polyurethane (PU), rubber, thermoplastic polyurethane (TPU), silicone or any combinations thereof.

3. The delivery device of any one of the preceding claims, wherein the guest polymer comprises a gel selected from hydrogel, aerogel, xerogel or any combinations thereof, preferably the guest polymer comprises poly(2-hydroxyethyl methacrylate) (PHEMA), preferably the gel is a copolymer of poly(2-hydroxyethyl methacrylate) (PHEMA) and PEGMEA.

4. The delivery device of any one of the preceding claims, wherein the delivery membrane comprises a continuous matrix of the host polymer and a plurality of interconnected paths of the guest polymer, wherein the interconnected paths of the guest polymer preferably extend through the whole thickness of the membrane, preferably , the delivery membrane has an outer surface and a plurality of paths of the guest polymer coincide with said inner surface and/or said outer surface.

5. The delivery device of any one of the preceding claims, wherein the delivery membrane comprises one or more chemical compounds in the form of particles, such as cells, drugs, buffers, surfactants, fragrances, dyes, flavours, antioxidants, nutrients, hormones, catalysts or any combinations thereof.

6. The delivery device of any one of the preceding claims, wherein the cavity comprises and/or is adapted for being filled and/or refilled with one or more chemical compounds in the form of fluids and/or particles and/or particles dispersed or dissolved in a liquid, preferably the particles being selected from cells, catalysts, drugs, buffers, surfactants, fragrances, dyes, flavours or any combinations thereof, the cavity is preferably adapted for being filled with one or more chemical compounds by injecting via an inlet comprising a valve, such as an inflation lumen and/or by injection via the delivery membrane.

**7.** The delivery device of any one of claims 5-6, wherein the cavity comprises particles comprising cells, such as stem cells or microorganisms, the cells are encapsulated in the cavity of the membrane and are preferably capable of producing at least one by-product which by-product is capable of migrating through the membrane, preferably via pathways of the guest polymer.

**8.** The delivery device of any one of claims 5-7, wherein the chemical substance comprises drug trapped in liposomes, preferably the liposomes are dispersed in a polar liquid, such as in an aqueous liquid, preferable the chemical substance comprises the drug trapped in liposomes together with metal particles, preferably gold particles, more preferably the chemical substance comprises the drug trapped in liposomes together with the metal particles and together with light emitting units which light emitting units can be triggered to emit light to heat the metal particles.

**9.** The delivery device of any one of the preceding claims, wherein the delivery device comprises a coating on at least a part of its inner and/or outer surface where the coating comprises a material identical to the guest polymer.

**10.** The delivery device of any one of the preceding claims, wherein the delivery device is a balloon catheter comprising at least one balloon forming the cavity, preferably the delivery device is a urinary catheter, such as a Foley catheter and the delivery membrane is the balloon or a section thereof.

**11.** The delivery device of claim 10, wherein the catheter comprises a diagnostic probe, said probe is adapted for detection of at least one biomarker such as, but not limited to, pH value, temperature, moisture level or infection level and combinations thereof (multiplexing), preferably the diagnostic probe is configured for changing upon a change of the at least one biomarker, said change of the probe is preferably optically readable, more preferably the probe is incorporated or mounted in a distal end of the catheter, such as in a distal tip of the catheter.

**12.** The delivery device of any one of claims 10-11, wherein the catheter comprises a read-out structure for reading the probe and transmitting the read signal to a displaying element for visually or audibly displaying, wherein the read-out structure comprises an optical or electrochemical reader, the read-out structure preferably comprises an optical read-out structure, wherein the catheter comprises an inflation and/or a drainage tube and a distal tip, said drainage tube comprises a channel for draining urine, said channel extending to the tip and said tip comprises or carries said probe, wherein said channel is configured for functioning as a waveguide when filled with liquid.

**13.** The delivery device of any one of claims 1 to 9, wherein the delivery device is a ring-shaped delivery device and the cavity is a ring-shaped or semi-ring-shaped cavity within the ring-shaped delivery device, preferably the delivery device is a contraceptive delivery device comprising a drug for preventing pregnancy or the delivery device contains in its cavity a drug for treating symptoms of alzheimers, such as T3D-959, donepezil, galantamine, memantine, rivastigmine or any combinations thereof.

**14.** The delivery device of any one of claims 1 to 9, wherein the delivery device is a capsule, such as a capsule having a volume of at least about 0.01 cm$^3$, such as a volume of at least about 0.05 cm$^3$, such as a volume of at least about 1 cm$^3$, such as a volume of at least about 5 cm$^3$, preferably the capsule is spherical, oblong, egg shaped, oval or flattened.

**15.** The delivery device of claim 14, wherein the capsule contains in its cavity a drug for treating symptoms of alzheimers, such as T3D-959, donepezil, galantamine, memantine, rivastigmine or any combinations thereof, preferably the drug is trapped in liposomes.

**Patentansprüche**

**1.** Abgabevorrichtung, geeignet zum Abgeben einer chemischen Substanz, die eine oder mehrere chemische Verbindungen umfasst, wobei die Abgabevorrichtung einen geschlossenen Hohlraum umfasst, wobei der Hohlraum durch eine innerste Wandfläche definiert ist, wobei zumindest ein Abschnitt der inneren Wandfläche eine innere Fläche einer Abgabemembran darstellt, wobei die Abgabemembran ein interpenetrierendes Polymernetzwerksubstrat umfasst, umfassend ein Wirtspolymer und ein Gastpolymer, wobei das Gastpolymer das Wirtspolymer interpenetriert, um fortlaufende Bahnen innerhalb des Wirtspolymers zu bilden, wobei die Abgabemembran bevorzugt eine äußere Fläche aufweist, die der inneren Fläche der Abgabemembran gegenüberliegt, wobei die fortlaufende Bahnen des Gastpolymers innerhalb des Wirtspolymers von der inneren Fläche zu der äußeren Fläche der Abgabemembran verlaufen.

2. Abgabevorrichtung nach Anspruch 1, wobei das Wirtspolymer ein vernetztes Polymer ist, wie etwa ein vernetztes Elastomer, z. B. ein thermoplastisches Elastomer (TPE), ein Polyolefin-Elastomer (POE), Polyurethan (PU), Kautschuk, ein thermoplastisches Polyurethan (TPU), Silikon oder beliebige Kombinationen davon.

3. Abgabevorrichtung nach einem der vorhergehenden Ansprüche, wobei das Gastpolymer ein Gel umfasst, ausgewählt aus Hydrogel, Aerogel, Xerogel oder beliebigen Kombinationen davon, wobei das Gastpolymer bevorzugt Poly(2-hydroxyethyl-methacrylat) (PHEMA) umfasst und das Gel bevorzugt ein Copolymer von Poly(2-hydroxyethyl-methacrylat) (PHEMA) und PEGMEA ist.

4. Abgabevorrichtung nach einem der vorhergehenden Ansprüche, wobei die Abgabemembran eine kontinuierliche Matrix des Wirtspolymers und eine Vielzahl von miteinander verbundenen Bahnen des Gastpolymers umfasst, wobei die miteinander verbundenen Bahnen des Gastpolymers bevorzugt durch die gesamte Dicke der Membran verlaufen, wobei bevorzugt die Abgabemembran eine äußere Fläche aufweist und eine Vielzahl von Bahnen des Gastpolymers mit der inneren Fläche und/oder der äußeren Fläche übereinstimmt.

5. Abgabevorrichtung nach einem der vorhergehenden Ansprüche, wobei die Abgabemembran eine oder mehrere chemische Verbindungen in der Form von Partikeln umfasst, wie etwa Zellen, Arzneimittel, Puffer, Tenside, Duftstoffe, Farbstoffe, Aromen, Antioxidantien, Nährstoffe, Hormone, Katalysatoren oder beliebige Kombinationen davon.

6. Abgabevorrichtung nach einem der vorhergehenden Ansprüche, wobei der Hohlraum eine oder mehrere chemische Verbindungen in der Form von Fluiden und/oder Partikeln und/oder Partikeln, die in einer Flüssigkeit dispergiert oder aufgelöst sind, umfasst und/oder dazu angepasst ist, damit befüllt und/oder wiederbefüllt zu werden, wobei die Partikel bevorzugt aus Zellen, Katalysatoren, Arzneimitteln, Puffern, Tensiden, Duftstoffen, Farbstoffen, Aromastoffen oder beliebige Kombinationen davon ausgewählt sind, wobei der Hohlraum bevorzugt dazu angepasst ist, mit einer oder mehreren chemischen Verbindungen durch Injizieren über einen Einlass, der ein Ventil umfasst, wie etwa ein Aufblaslumen und/oder durch Injektion über die Abgabemembran, befüllt zu werden.

7. Abgabevorrichtung nach einem der Ansprüche 5-6, wobei der Hohlraum Partikel umfasst, die Zellen umfassen, wie etwa Stammzellen oder Mikroorganismen, wobei die Zellen in dem Hohlraum der Membran eingekapselt sind und bevorzugt in der Lage sind, zumindest ein Nebenprodukt herzustellen, wobei das Nebenprodukt in der Lage ist, durch die Membran zu migrieren, bevorzugt über Bahnen des Gastpolymers.

8. Abgabevorrichtung nach einem der Ansprüche 5-7, wobei die chemische Substanz ein in Liposomen eingeschlossenes Arzneimittel umfasst, wobei die Liposomen in einer polaren Flüssigkeit wie etwa einer wässrigen Flüssigkeit dispergiert sind, wobei die chemische Substanz das Arzneimittel in Liposomen zusammen mit Metallpartikeln, bevorzugt Goldpartikeln, eingeschlossen umfasst, wobei die chemische Substanz das Arzneimittel bevorzugter in Liposomen zusammen mit den Metallpartikeln und zusammen mit lichtemittierenden Einheiten eingeschlossen umfasst, wobei die lichtemittierenden Einheiten dazu ausgelöst werden können, Licht zu emittieren, um die Metallpartikel zu erwärmen.

9. Abgabevorrichtung nach einem der vorhergehenden Ansprüche, wobei die Abgabevorrichtung eine Beschichtung an zumindest einem Teil ihrer inneren und/oder äußeren Fläche umfasst, wobei die Beschichtung ein Material umfasst, das mit dem Gastpolymer identisch ist.

10. Abgabevorrichtung nach einem der vorhergehenden Ansprüche, wobei die Abgabevorrichtung ein Ballonkatheter ist, umfassend zumindest einen Ballon, der den Hohlraum bildet, wobei die Abgabevorrichtung bevorzugt ein Harnkatheter wie etwa ein Foley-Katheter ist und die Abgabemembran der Ballon oder ein Abschnitt davon ist.

11. Abgabevorrichtung nach Anspruch 10, wobei der Katheter eine Diagnosesonde umfasst, wobei die Sonde zur Erkennung zumindest eines Biomarkers wie etwa unter anderem pH-Wert, Temperatur, Feuchtigkeitspegel oder Infektionspegel und Kombinationen davon (Multiplexing) angepasst ist, wobei die Diagnosesonde bevorzugt zum Ändern bei einer Änderung zumindest eines Biomarkers konfiguriert ist, wobei die Änderung der Sonde bevorzugt optisch ablesbar ist, wobei die Sonde bevorzugter in einem distalen Ende des Katheters, wie etwa in der distalen Spitze des Katheters, integriert oder daran befestigt ist.

12. Abgabevorrichtung nach einem der Ansprüche 10-11, wobei der Katheter eine Auslesestruktur zum Auslesen der Sonde und zum Übertragen des Auslesesignals an ein Anzeigeelement zur visuellen oder akustischen Darstellung umfasst, wobei die Auslesestruktur eine optische oder elektrochemische Lesevorrichtung umfasst, wobei die Auslesestruktur bevorzugt eine optische Auslesestruktur umfasst, wobei der Katheter einen Aufblas- und/oder einen Drainagetubus und eine distale Spitze umfasst, wobei der Drainagetubus einen Kanal zum Ablassen von Urin umfasst, wobei der Kanal zu der Spitze verläuft und die Spitze die Sonde umfasst oder trägt, wobei der Kanal zum Betrieb als ein Wellenleiter, wenn er mit Flüssigkeit gefüllt ist, konfiguriert ist.

13. Abgabevorrichtung nach einem der Ansprüche 1 bis 9, wobei die Abgabevorrichtung eine ringförmige Abgabevorrichtung ist und der Hohlraum ein ringförmiger oder halbringförmiger Hohlraum innerhalb der ringförmigen Abgabevorrichtung ist, wobei die Abgabevorrichtung bevorzugt eine Abgabevorrichtung zur Empfängnisverhütung ist, umfassend ein Arzneimittel zur Vorbeugung einer Schwangerschaft, oder wobei die Abgabevorrichtung in ihrem Hohlraum ein Arzneimittel zum Behandeln von Symptomen von Alzheimer-Krankheit enthält, wie etwa T3D-959, Donepezil, Galantamin, Memantin, Rivastigmin oder beliebige Kombinationen davon.

14. Abgabevorrichtung nach einem der Ansprüche 1 bis 9, wobei die Abgabevorrichtung eine Kapsel ist, wie etwa eine Kapsel mit einem Volumen von zumindest etwa 0,01 cm$^3$, wie etwa einem Volumen von zumindest etwa 0,05 cm$^3$, wie etwa einem Volumen von zumindest etwa 1 cm$^3$, wie etwa einem Volumen von zumindest etwa 5 cm$^3$, wobei die Kapsel bevorzugt kugelförmig, länglich, eiförmig, oval oder abgeflacht ist.

15. Abgabevorrichtung nach Anspruch 14, wobei die Kapsel in ihrem Hohlraum ein Arzneimittel zum Behandeln von Symptomen von Alzheimer-Krankheit enthält, wie etwa T3D-959, Donepezil, Galantamin, Memantin, Rivastigmin oder beliebige Kombinationen davon, wobei das Arzneimittel bevorzugt in Liposomen eingeschlossen ist.

## Revendications

1. Dispositif d'administration approprié pour administrer une substance chimique comprenant un ou plusieurs composés chimiques, le dispositif d'administration comprenant une cavité fermée, la cavité étant définie par une surface de paroi la plus à l'intérieur, au moins une section de la surface de paroi la plus à l'intérieur constituant une surface interne d'un membrane d'administration, ladite membrane d'administration comprenant un substrat de réseau polymère interpénétrant comprenant un polymère hôte et un polymère invité, où le polymère invité interpénètre le polymère hôte pour former des chemins continus à l'intérieur dudit polymère hôte, de préférence ladite membrane d'administration comprend une surface externe opposée à ladite surface interne de ladite membrane d'administration, lesdits chemins continus dudit polymère invité à l'intérieur dudit polymère hôte s'étendant de la surface interne à la surface externe de ladite membrane d'administration.

2. Dispositif d'administration selon la revendication 1, ledit polymère hôte étant un polymère réticulé tel qu'un élastomère réticulé, par ex. un élastomère thermoplastique (TPE), un élastomère polyoléfinique (POE), un polyuréthane (PU), un caoutchouc, un polyuréthane thermoplastique (TPU), un silicone ou toutes les combinaisons de ceux-ci.

3. Dispositif d'administration selon l'une quelconque des revendications précédentes, ledit polymère invité comprenant un gel choisi parmi l'hydrogel, l'aérogel, le xérogel ou toutes les combinaisons de ceux-ci, de préférence ledit polymère invité comprenant le polyméthacrylate de 2-hydroxyéthyle (PHEMA), de préférence le gel est un copolymère de polyméthacrylate de 2-hydroxyéthyle (PHEMA) et le PEGMEA.

4. Dispositif d'administration selon l'une quelconque des revendications précédentes, ladite membrane d'administration comprenant une matrice continue du polymère hôte et une pluralité de trajets interconnectés du polymère invité, lesdits trajets interconnectés du polymère invité s'étendant de préférence sur toute l'épaisseur de la membrane, de préférence, la membrane d'administration comportant une surface externe et une pluralité de trajets du polymère invité coïncidant avec ladite surface interne et/ou ladite surface externe.

5. Dispositif d'administration selon l'une quelconque des revendications précédentes, ladite membrane d'administration comprenant un ou plusieurs composés chimiques sous la forme de particules, tels que des cellules, des médicaments, des tampons, des tensioactifs, des parfums, des colorants, des arômes, des antioxydants, des nutriments, des hormones, des catalyseurs ou toutes les combinaisons de ceux-ci.

6. Dispositif d'administration selon l'une quelconque des revendications précédentes, ladite cavité comprenant et/ou étant adaptée pour être remplie et/ou remplie de nouveau avec un ou plusieurs composés chimiques sous la forme de fluides et/ou de particules et/ou de particules dispersées ou dissoutes dans un liquide, de préférence les particules étant choisies parmi les cellules, les catalyseurs, les médicaments, les tampons, les tensioactifs, les parfums, les colorants, les arômes ou toutes les combinaisons de ceux-ci, la cavité étant de préférence adaptée pour être remplie d'un ou plusieurs composés chimiques par injection via une entrée comprenant un valve, telle qu'une lumière de gonflage et/ou par injection via la membrane d'administration.

7. Dispositif d'administration selon l'une quelconque des revendications 5 à 6, ladite cavité comprenant des particules comprenant des cellules, telles que des cellules souches ou des micro-organismes, les cellules étant encapsulées dans la cavité de la membrane et étant de préférence capables de produire au moins un sous-produit, lequel sous-produit étant capable de migrer à travers la membrane, de préférence via les chemins du polymère invité.

8. Dispositif d'administration selon l'une quelconque des revendications 5 à 7, ladite substance chimique comprenant un médicament piégé dans des liposomes, de préférence les liposomes étant dispersés dans un liquide polaire, tel qu'un liquide aqueux, de préférence la substance chimique comprenant le médicament piégé dans des liposomes conjointement avec des particules métalliques, de préférence des particules d'or, plus préférablement la substance chimique comprenant le médicament piégé dans des liposomes conjointement avec des particules métalliques et conjointement avec des unités luminescentes, lesquelles unités luminescentes pouvant être déclenchées pour émettre de la lumière pour chauffer les particules métalliques.

9. Dispositif d'administration selon l'une quelconque des revendications précédentes, ledit dispositif d'administration comprenant un revêtement sur au moins une partie de sa surface interne et/ou externe où le revêtement comprend un matériau identique au polymère invité.

10. Dispositif d'administration selon l'une quelconque des revendications précédentes, ledit dispositif d'administration étant un cathéter à ballonnet comprenant au moins un ballonnet formant la cavité, de préférence ledit dispositif d'administration étant un cathéter urinaire, tel qu'un cathéter de Foley et la membrane d'administration étant le ballonnet ou une section de celui-ci.

11. Dispositif d'administration selon la revendication 10, ledit cathéter comprenant une sonde de diagnostic, ladite sonde étant adaptée pour la détection d'au moins un biomarqueur tel que, mais sans s'y limiter, la valeur du pH, la température, le taux d'humidité ou le niveau d'infection et les combinaisons de ceux-ci (multiplexage), de préférence la sonde de diagnostic étant conçue pour changer lors d'un changement dudit au moins un biomarqueur, ledit changement de la sonde étant de préférence lisible optiquement, plus préférablement la sonde étant incorporée ou montée dans une extrémité distale du cathéter, telle que dans une pointe distale du cathéter.

12. Dispositif d'administration selon l'une quelconque des revendications 10 à 11, ledit cathéter comprenant une structure de lecture pour lire la sonde et transmettre le signal de lecture à un élément d'affichage pour un affichage visuel ou audible, ladite structure de lecture comprenant un lecteur optique ou électrochimique, ladite structure de lecture comprenant de préférence une structure de lecture optique, ledit cathéter comprenant un tube de gonflage et/ou de drainage et une pointe distale, ledit tube de drainage comprenant un canal pour drainer l'urine, ledit canal s'étendant jusqu'à la pointe et ladite pointe comprenant ou portant ladite sonde, ledit canal étant conçu pour fonctionner comme un guide d'ondes lorsqu'il est rempli de liquide.

13. Dispositif d'administration selon l'une quelconque des revendications 1 à 9, ledit dispositif d'administration étant un dispositif d'administration en forme d'anneau et ladite cavité étant une cavité en forme d'anneau ou de demi-anneau à l'intérieur du dispositif d'administration en forme d'anneau, de préférence ledit dispositif d'administration étant un dispositif d'administration de contraceptif comprenant un médicament pour prévenir la grossesse ou ledit dispositif d'administration contenant dans sa cavité un médicament pour traiter des symptômes de la maladie d'Alzheimer, tel que le T3D-959, le donépézil, la galantamine, la mémantine, la rivastigmine ou toutes les combinaisons de ceux-ci.

14. Dispositif d'administration selon l'une quelconque des revendications 1 à 9, ledit dispositif d'administration étant une capsule, telle qu'une capsule ayant un volume d'au moins environ 0,01 cm$^3$, tel qu'un volume d'au moins environ 0,05 cm$^3$, tel qu'un volume d'au moins environ 1 cm$^3$, tel qu'un volume d'au moins environ 5 cm$^3$, de préférence la capsule étant sphérique, oblongue, de forme ovoïde, ovale ou aplatie.

15. Dispositif d'administration selon la revendication 14, ladite capsule contenant dans sa cavité un médicament pour traiter des symptômes de la maladie d'Alzheimer, tel que le T3D-959, le donépézil, la galantamine, la mémantine, la rivastigmine ou toutes les combinaisons de ceux-ci, de préférence ledit médicament étant piégé dans des liposomes.

Methylene blue transmission through retention balloons of catheters

Fig. 1

Fig. 2

Fig. 3

Fig. 4

Fig. 5

Fig. 6

## Fig.7a

52a

51a

## Fig.7b

52b

51b

EP 3 334 413 B1

Fig.8a

Fig.8b

## Fig.9a

71a

72a

73b

EP 3 334 413 B1

## Fig.9b

72b

71b

73b

## Fig.10

Silicone          IPN          Hydrated IPN

Fig.11

Fig.12

# Fig.13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005055972 A
- CN 103623497 A
- US 5569184 A
- CN 101733051
- US 2014309598 A
- US 2012080378 A

- US 2015038613 A
- WO 2013075724 A
- US 2014303263 A
- US 7498177 B
- US 6602243 B
- WO 2010042869 A

### Non-patent literature cited in the description

- **GARCIA et al.** 5-Fluorouracil trapping in poly(2-hydroxyethyl methacrylate-co-acrylamide) hydrogels; in vitro drug delivery studies. *European Polymer Journal,* 2000, vol. 36, 111-122
- **FERREIRA et al.** Evaluation of poly(2-hydroxyethyl methacrylate) gels as drug delivery systems at different pH values. *International Journal of Pharmaceuticals,* 2000, vol. 194, 169-180
- **KARLGARD et al.** In vitro uptake and release studies of ocular pharmaceutical agents by silicon-containing and p-HEMA hydrogel contact lens materials. *International Journal of Pharmaceuticals,* 2003, vol. 257, 141-151

- **SANTOS et al.** Poly(hydroxyethyl methacrylate-co-methacrylated-p-cyclodextrin) hydrogels: Synthesis, cytocompatability, mechanical properties and drug loading/release properties. *ScienceDirect, Acta Biomaterialia,* 2008, vol. 4, 745-755
- Wireless Optofluidic Systems for Programmable In Vivo Pharmacology and Optogenetic. **JAE-WOONG JEONG et al.** Cell. Elsevier Inc, 30 July 2015, vol. 162, 662-674